# EUROPÄISCHE PATENTSCHRIFT

(11) **EP 2 546 290 B1**
(45) Veröffentlichungstag und Bekanntmachung des Hinweises auf die Patenterteilung: **07.09.2016**
(21) Anmeldenummer: 12007134.5
(22) Anmeldetag: 22.07.2005
(51) Int. Cl.: C08J 7/04, A61M 16/06

(54) **Patienteninterface mit Beschichtung**
Patient interface with coating
Dispositif d'interface de patient doté d'un revêtement

(30) Priorität: 03.09.2004 DE 202004043208 U; 18.03.2005 DE 202005013079 U; 14.06.2005 DE 202005027724 U
(43) Veröffentlichungstag der Anmeldung: 16.01.2013
(62) Teilanmeldung aus: 05771006.3
(73) Patentinhaber: Löwenstein Medical Technology GmbH + Co. KG, 22525 Hamburg (DE)
(72) Erfinder: Feldhahn, Karl-Andreas, Dr., 22761 Hamburg (DE); Eifler, Martin, 25348 Glückstadt (DE); Frerichs, Arnold, 21614 Buxtehude (DE); Marx, Thomas, Dr., 22525 Hamburg (DE)
(74) Vertreter: Marx, Thomas

(56) Entgegenhaltungen:
- EP-A1- 1 694 153
- EP-A2- 0 298 743
- WO-A1-2004/052640
- US-A- 5 405 666

## Beschreibung

Die Erfindung betrifft Kunststoffe für medizintechnische Geräte. Ferner betrifft die Erfindung Formkörper zur Bildung von medizintechnischen Geräten sowie Verfahren zur Herstellung von erfindungsgemäßen Kunststoffen und/oder Formkörpern.

Medizintechnische Geräte können eine bakterielle Belastung beim Nutzer verursachen. Möglichen Quellen sind: die Produktion, der Service, die Klinik und der Patient/Nutzer. Nach dem Stand der Technik werden medizintechnische Geräte daher einer Desinfektion unterzogen und/oder autoklaviert.

Die WO 2004/052640 A1 offenbart einen Artikel mit einer funktionalen Beschichtung, die Mikropartikel aufweist und die wasserabweisend ist oder Antifouling Eigenschaften aufweist. Die Mikropartikel sind beispielsweise Silikonoxid. Die Beschichtung ist organisch und weist Flouraklkyl oder Alkylgruppen auf oder ist organisch und weist Polyalkyleneoxylgruppen auf.

Die EP0298743 A2 offenbart eine Beschichtung für Silikongele, die bei der Herstellung klebend sind, was deren Weiterverarbeitung erschwert. Die Silikongele werden daher mit einer Beschichtung aus gehärteten Silikonkautschukkügelchen beschichtet. Der Durchmesser dieser Silikonkautschukkügelchen ist bei 0,1 bis 1000 µM.

Die US 5,405,666 A offenbart beschichtete Elastomerartikel für den medizinischen Einsatz, die durch die Beschichtung eine verbesserte Gleitfähigkeit insbesondere an der Haut aufweisen. Als elastomere Formkörper werden Handschuhe, Kondome, Katheter, Urether genannt. Der Formkörper besteht aus Polyurethan, Polyacrylat, Polybutylen, Naturkautschuk oder Silikonkautschuk. Auf den Formkörper werden Mikropartikel aus Siliziumdioxid aufgebracht. Diese werden mittels einer Oberflächenbeschichtung, die als Bindemittel für die Mikropartikel dient, an der Oberfläche des Formkörpers fixiert. Dem Siliziumdioxid wird für Anwendung bei Handschuhen ein Tensid beigemischt, welches auch bakterizide Eigenschaften haben kann. Als Bindemittel für die Oberflächenbeschichtung werden Polyurethan oder Vinylacetat vorgeschlagen. Die beschichtete Elastomerartikel der US 5,405,666 A sind Einmalartikel, die nicht wiederverwendet werden.
Der Maskenwulst von Beatmungsmasken hingegen ist ein Mehrwegartikel der durchaus ein Jahr oder länger in Gebrauch ist. Da Beatmungsmasken von Patienten mehrere Stunden täglich getragen werden, ist eine häufige Reinigung oder eine hygienische Aufbereitung des Maskenwulstes notwendig.
Ein gut geeignetes Material für Maskenwülste ist Silikon. Jedoch hat Silikon den Nachteil, für den Patienten unangenehm auf der Haut anzuhaften. Dies gilt insbesondere wenn die Haut leicht fettig ist. Zudem haften Fett und Schmutz gut an der Silikonoberfläche.

Es ist daher Aufgabe der Erfindung, eine Oberflächenbeschichtung für einen Maskenwulst anzugeben, die die Oberfläche von Silikonwülsten derart modifiziert, dass die Haftneigung der Silikonoberfläche an der Haut vermindert und die Anhaftneigung von Hautfett und Schmutz vermindert beziehungsweise die Reinigung des Maskenwulstes erleichtert wird. Zudem ist es die Aufgabe der Erfindung die ansonsten positiven Eigenschaften des Silikonwulstes nicht durch die Oberflächenbeschichtung zu beeinträchtigen und die Oberflächenbeschichtung sicher und dauerhaft, und weitgehend ohne die Verwendung von Haftvermittlern, mit der Silikonunterlage zu verbinden.

Diese Aufgabe wird erfindungsgemäß dadurch gelöst, daß der Formkörper der Maskenwulst einer Beatmungsmaske ist wobei der Maskenwulst aus Silikon hergestellt ist und die Mikropartikel, die die Erhebungen auf der Oberfläche bilden, ausgewählt sind aus Silikaten, Mineralien, Metalloxiden, Metallpulvern, Kieselsäuren, Pigmenten oder Polymeren und die Oberflächenbeschichtung aus Silikon hergestellt ist.

Darüber hinaus besteht eine zusätzliche Aufgabe der vorliegenden Erfindung darin, ein entsprechendes Herstellungs-Verfahren für erfindungsgemäße Maskenwülste anzugeben, sodass eine funktionelle und zugleich preiswerte sowie langfristig funktionsfähige Ausführungsform bereitgestellt wird.

Diese Aufgabe wird erfindungsgemäß dadurch gelöst, daß der Formkörper mittels Kunststoffspritzguß hergestellt und anschließend mindestens bereichsweise beschichtet wird.

Grundsätzlich wird durch die Verwendung von oberflächenbeschichteten Komponenten in medizintechnischen Geräten ein äußerst weiter konstruktiver Freiraum eröffnet. Die jeweils gewünschten funktionellen Eigenschaften können durch die Oberflächenbeschichtung unabhängig vom Material des Grundkörpers bereitgestellt werden. Die funktionellen Eigenschaften können beispielsweise die bereits erwähnten keimtötenden Eigenschaften sein, die funktionellen Eigenschaften können sich aber auch auf Gleiteigenschaften, Reibungseigenschaften, Oberflächengestaltungen oder Oberflächenhärten beziehen.

Entsprechend der jeweils vorgegebenen funktionellen Eigenschaft wird die Oberflächenbeschichtung des Grundkörpers gewählt und das Basismaterial des Grundkörpers kann unabhängig von der gewünschten funktionellen Eigenschaft der Oberfläche und der mechanischen oder statischen Randbedingungen festgelegt werden. Beispielsweise ist es möglich, bei hohen mechanischen Belastungen einen harten Grundkörper mit einer weicheren Oberflächenbeschichtung zu versehen oder einen weichen und elastischen Grundkörper mit einer keimabweisenden funktionellen Oberfläche auszustatten. Gegebenenfalls werden die gewünschten Oberflächenbeschichtungen unter Verwendung geeigneter Zwischenschichten als Haftvermittler auf die Grundkörper aufgebracht.

Der Formkörper ist insbesondere für Geräteteile, Geräteoberschalen, Geräteinnenteile, Gerätezubehörteile, Gerätekomponenten, Abformmaterialien, Füllungsmaterialien für medizintechnische Geräte und/oder medizinische Produkte ausgebildet und dadurch gekennzeichnet, dass der Formkörpers wenigstens abschnittsweise Keimansiedelungen abweisend ausgebildet ist, sodass ein Ansiedelung von Keimen verhindert wird.

Der Formkörper kann im wesentlichen als Geräteteil, Geräteoberschale, Geräteinnenteil, Gerätezubehörteil, Gerätekomponente, Luftanfeuchter, Vernebler, Medikamentenzerstäuber, Beatmungsgerät, Lufteingangsfilter, Schalldämpfer, Luftweg im Gerät, Filter, Beatmungsmaske, Beatmungsschlauch, Notfallbeatmungsgerät, Absaugeinrichtung, Absaugschlauch, Auffangbehälter von einer Absaugeinrichtung oder für als Defibrillatorgehäuse oder -gehäuseteil ausgebildet, sein.

Vorzugsweise ist ein derartiger Formkörper eines medizintechnischen Geräts wenigstens teilweise und/oder abschnittsweise aus Kunststoff gefertigt. Dabei werden häufig verschiedene Kunststoffe verwendet. Die Kunststoffe erfüllen unterschiedliche Funktionen und müssen für die auszuübende Funktion bestmöglich geeignet sein. So sind die Kunststoffe für beispielsweise ein Beatmungsgerät mit den Komponenten Eingangsfilter - Beatmungsgerät - Ausgangsfilter - Patientenschlauch - Filter - Patientenkontaktstelle jeweils für den Einsatzzweck optimiert.

Als Kunststoffe sind alle bekannten Kunststoffe denkbar. Bspw. seien hier genannt: Polyethylene, Polypropylene, Polyvinylchloride, Polystyrole, Polycarbonate, Cellophane, Celluloseacetate, Polyelofine, fluorierte Kohlenwasserstoffe (Teflon), Polyhydroxyethylmethacrylate (PHEMA) (Hydron), Polymethylmethacrylate (PMMA), Polysiloxane, Polyether, Polyester, Polyacetale, Polyvinyle, Polyethersilikone, Polyurethane, Natürliches und synthetisches Gummi, Silikon, Latex, ABS-Harz, Acrylharze, Triacetate, Vinylide und Reyon.

Ferner können alle zum Spritzgießen von Spritzgusskörpern geeigneten Polymere eingesetzt werden. Bevorzugt werden als Materialien für das Spritzgießen Polymere oder Polymerblends eingesetzt, die ein Polymer auf der Basis von Polycarbonaten, Polyoxymethylenen, Poly(meth)acrylaten, Polyamiden, Polyvinylchlorid, Polyethylenen, Polypropylenen, aliphatischen linearen- oder verzweigten Polyalkenen, cyclischen Polyalkenen, Polystyrolen, Polyestern, Polyethersulfonen, Polyacrylnitril oder Polyalkylenterephthalaten, Poly(vinylidenfluorid), Poly(hexafluorpropylen), Poly(per-fluorpropylenoxid), Poly(fluoralkylacrylat), Poly(fluoral-kylmethacrylat), Poly(vinylperfluoralkylether) oder andere Polymere aus Perfluoralkoxyverbindungen, Poly(isobuten), Poly(4- methyl-1-penten), Polynorbonen als Homo- oder Copolymer oder deren Gemische, aufweisen. Ganz besonders bevorzugt werden als Material für das Spritzgießen Polymere oder Polymerblends eingesetzt, die ein Polymer auf Basis von Poly (ethylen), Poly(propylen), Polymethylmethacrylaten, Polystyrolen, Polyestern, AcrylnitrilButadien-Styrol Terpolymere (ABS) oder Poly(vinylidenfluorid) aufweisen, wobei die Kunststoffe in Reinform und/oder als Gemisch verwandt werden können.

Neben Kunststoffen können auch Metalle und/oder Keramik und/oder Glas oder beliebige Kombination dieser Materialien, auch mit den oben genannten Kunststoffen, Anwendung finden.

Der Formkörper kann wenigstens teilweise aus einem keimabweisenden Material gefertigt sein. Hierbei wird unter keimabweisend antibakteriell, antimikrobiell, antiviral, fungizid, germizid oder wachstumshemmend verstanden.

Vorzugsweise ist jedoch vorgesehen, dass der Formkörper eine wenigstens abschnittsweise keimabweisend ausgebildete Oberfläche aufweist. Bevorzugt ist es vorgesehen, dass die Oberfläche des Formkörpers wenigstens abschnittsweise durch Verringerung der Oberflächenadhäsion keimabweisend ausgebildet ist. Bakterien und andere Mikroorganismen benötigen zur Adhäsion an einer Oberfläche oder zur Vermehrung an einer Oberfläche Wasser, welches an hydrophoben Oberflächen nicht zur Verfügung steht. Die erfindungsgemäße Oberfläche verhindert das Anwachsen von Bakterien und anderen Mikroorganismen und ist somit bakteriophob und/oder antimikrobiell.

Ein Ansatz ist die Verringerung der Adhäsion von Proteinen auf Oberflächen. Da Zellmembranen von Mikroorganismen gleichfalls aus Proteinbestandteilen aufgebaut sind, wird durch die Adhäsionsverringerung von Proteinen die Anlagerung von Mikroorganismen reduziert.

Hierzu können Polymere aus einem oder mehreren radikalisch polymerisierbaren Monomeren verwendet werden, wobei diese Polymere Gruppen mit permanenter positiver Ladung und Gruppen, die zur Ausbildung einer Bindung an Oberflächen befähigt sind, besitzen.

Alternativ können zwitterionische Gruppen enthaltende Polymere verwendet werden. Die zwitterionische Gruppe ist üblicherweise ein Ammoniumsalz-Ester. Das Polymer, welches diese Gruppe enthält, kann über die freie radikalische Polymerisation von ethylenisch ungesättigten Monomeren zusammen mit einem Monomer, welches eine zwitterionische Gruppe enthält, hergestellt werden.

Ferner können Phosphorylcholingruppen sowie nicht-ionischen ethoxylierten und propoxylierten Surfaktanten verwendet werden. Außerdem können biologische Polymere und synthetische Surfaktanten verwendet werden, um die Adhäsion gewisser Bakterienspezies durch die Erzeugung hydrophober Oberflächen zu verringern.

Es können auch antiadhäsive Oberflächen durch Beschichtungen mit Hyaluronderivaten verwendet werden oder Polymere mit Wiederholungseinheiten mit polaren oder polarisierbaren, modulierenden und hydrophoben Fluorid-enthaltenden Gruppen.

Besonders vorteilhaft ist es, speziell modifizierte Kunststoffe zu verwenden, die adhäsionsverringernde Substanzen in einer solchen Menge aufweisen, dass die Adhäsion von Mikroorganismen auf deren Oberfläche um mindestens 50% geringer ist als bei nicht-modifizierten Kunststoffen, und/oder dass sie biozide Substanzen in einer solchen Menge aufweisen, dass mindestens 60% der verbleibenden Mikroorganismen innerhalb von 24 Stunden abgetötet werden.

Es ist auch vorgesehen, dass die Oberfläche des Formkörpers durch Abtötung und/oder Wachstumshemmung von Mikroorganismen keimabweisend ausgebildet ist. Zur Abtötung oder Wachstumshemmung können antibakterielle, antimikrobielle, anti-virale, fungizide, germizide, oder wachstumshemmende Substanzen aus allen geeigneten chemischen Gruppen verwendet werden.

Als adhäsionsverringernde Substanzen und Methoden zur Herstellung adhäsionsverringerter Kunststoffe bzw. Oberflächen eignen sich beispielsweise solche auf Phosphorylcholinbasis oder Phosphorylethanolaminbasis oder auch solche auf der Basis von Polyestern aus Einheiten, die von Glycerophosphorylcholin oder Glycerophosphorylethanolamin abgeleitet sind und polyfunktionellen Säuren oder deren Derivaten. Die adhäsionsverringernden Substanzen werden in Mengen von ca. 0,05 bis 50 Gew.-Teilen, vorzugsweise 0,1 bis 20 Gew.-Teilen, pro 100 Gew.-Teile Gesamtmasse eingebracht oder als Oberflächenbeschichtung aufgebracht.

Die modifizierten Formkörper für medizintechnische Geräte weisen adhäsionsverringernde Substanzen in einer solchen Menge auf, dass die Adhäsion von Mikroorganismen auf deren Oberfläche um mindestens 50% geringer ist als bei nicht-modifizierten Kunststoffen, und/oder dass sie biozide Substanzen in einer solchen Menge aufweisen, dass mindestens 60% der verbleibenden Mikroorganismen innerhalb von 24 Stunden abgetötet werden.

Diese modifizierten Formkörper haben vorzugsweise adhäsionsverringernde Substanzen inkorporiert. Ferner sind deren Oberflächen durch adhäsionsverringernde Substanzen modifiziert. Außerdem sind vorzugsweise biozide Substanzen inkorporiert, wobei als biozide Substanzen Silber, Silberionen freisetzende Substanzen, Kupfer, Kupferionen freisetzende Substanzen, Zink oder Zinkionen freisetzende Substanzen inkorporiert wurden.

Zur Herstellung modifizierter Kunststoffe werden die adhäsionsverringernden und bioziden Substanzen in die Kunststoffe eingebracht. In einer weiteren, bevorzugten Weiterbildung ist vorgesehen, dass die Oberfläche des Formkörpers als Wirkstoff Silber und/oder silberhaltige Verbindungen aufweist.
Beispielsweise ist IONPURE (TM) ein Nanosilber-Additiv, welches Kunststoff-Produkten, Kunstfasern, Kompositen, Silikonprodukten, Lacken und Beschichtungen antibakterielle und fungizide Eigenschaften verleiht. Silberionen in einer Glasmatrix sind die Wirkkomponenten. Im Gegensatz zu Standard-Antibiotika sind Bakterien nicht in der Lage, Resistenzen gegen Silberionen zu bilden. Die Einbindung von Silberionen in einer Glasmatrix verhindert unerwünschte Reaktionen, bekannt von anderen Additiven' auf Basis von Silberionen, die zum Vergrauen führen.

Die erforderliche Menge von 0,2 - 0,5% IONPURE hat unwesentliche Auswirkungen auf die physikalischen Eigenschaften der Kunststoffprodukte. Die antimikrobielle Wirkung ist über Jahre hinweg sichergestellt.

Alternativ können kleinste Silberteilchen in den Hohlräumen der Molekülketten von Polymeren verteilt werden. Die Elastizität wird hierbei nicht beeinträchtigt, die biologische Verträglichkeit des Materials ist gewahrt, und die Fähigkeit, Keime abzutöten, bleibt über viele Monate erhalten.

Wenn als Wirkstoff der antimikrobiellen Kunststoffe Silberionen verwendet werden, kann als Trägermaterial der Silberionen ein Zeolith verwendet werden, ein natürlich vorkommendes, extrem stabiles Mineral. Das Kristallgitter des Zeoliths besitzt große Porenvolumina, so können viele Silberionen gebunden werden. Durch Ionenaustauschprozesse setzt das Zeolith die Silberionen frei und bindet dafür Kationen der Umgebung wie Natrium, Kalium und Calcium. Durch diese kontinuierliche Freisetzung der Silberionen wird eine hohe und langanhaltende Wirkung der antimikrobiellen Kunststoffe erzielt. Der Wirkstoff ist beständig gegen Chemikalien mit pH-Werten von 3 bis 10 sowie gegen Temperaturen bis zu 800°C. Dass das Compound antibakteriell und wachstumshemmend gegenüber Bakterien, Hefen, Schimmel und Pilzen wirkt, konnte bei mehr als 25 Mikrobenstämmen nachgewiesen werden. Dazu gehören z.B. Kolibakterien, Salmonellen und Staphylokokken.

Ein weiteres antibakteriell wirksames Polymere auf Basis nanoskaliger Silberpartikel ist "Polygiene(TM)" (Perstorp, Schweden); eine duroplastische Formmasse mit antiviralen und antibakteriellen Eigenschaften.

Silberpartikel können in Größen bis hinab zu fünf Nanometer Durchmesser hergestellt werden. Dafür wird das Edelmetall im Vakuum verdampft und auf einer Oberfläche wieder kondensiert. Normalerweise erreicht man so Partikelgrößen zwischen 50 und 100 Nanometern, auf fließenden Flüssigkeitsfilmen sind jedoch auch fünf Nanometer erreichbar. Die kondensierten Partikel sind porös und wegen ihrer großen Oberfläche besonders aktiv.

Das Silber ist außerdem hochrein und nicht wie bei chemischen Produktionsprozessen mit anderen Stoffen verunreinigt. Die Nanopartikel werden fein verteilt in Polymere eingearbeitet oder als dünne Beschichtung aufgebracht.

Das Nanosilber wird im Batch verteilt. Im fertigen Produkt wird Nanosilber von der Oberfläche, durch Gebrauch, weggewischt. Silber wird fortlaufend durch Wasser an die Oberfläche gebracht. Die antibakteriellen Eigenschaften bleiben über Monate erhalten.

In einer weiteren, bevorzugten Ausführungsform ist vorgesehen, dass die Oberfläche als Wirkstoff ein antimikrobiell wirksames Polymere aufweist.

Bevorzugt werden zur Herstellung der antimikrobiellen Polymere Stickstoff und Phosphorfunktionalisierte Monomere eingesetzt, insbesondere werden diese Polymere aus mindestens einem der folgenden Monomere hergestellt: Methacrylsäure-2-tert.-butylaminoethylester, Methacrylsäure-2-diethylamino-ethylester, Methacrylsäure-2-diethylaminomethylester, Acrylsäure-2-tert.-butylaminoethylester, Acrylsäure-3-dimethylaminopropylester, Acrylsäure-2-diethylaminoethyl-ester, Acrylsäuredimethylaminoethylester, Dimethylaminopropylmethacrylamid, Diethylaminopropylmethacrylamid, Acrylsäure-3-dimethylaminopropylamid, 2-Methacryloyloxethyltrimethylammoniummethosulfat, Methacrylsäure-2-diethylaminoethylester, 2-Methacryloyloxyethyltrimethylammoniumchlorid, 3-Methacryloylaminopropyltrimethylammonium- chlorid, 2-Methacryloyloxyethyltrimethylammoniumchlorid, 2-Acryloyloxyethyl-4-benzoydimethylammoniumbromid,2-Methacryloyloxyethyl-4-benzoyldimethylammoniumbromid, Allyltriphenylphosphoniumbromid, Allyltriphenylphosphoniumchlorid, 2-Acrylamido-2-methyllpropansulfonsäure,2-Diethylaminoethylvinylether, 3-Aminopropylvinylether.

Die antimikrobiell zu versehende Oberfläche kann dabei vollständig oder teilweise mit den Polymeren bedeckt sein.

Es ist möglich, das antimikrobielle Polymere nur anzuschmelzen und/oder nur so wenig auf die Oberfläche aufzubringen, das ggf. eine Kornstruktur des antimikrobiellen Polymeren erhalten bleibt. Es wird so eine Strukturierung der Oberfläche erreicht.

Es ist weiter vorgesehen, dass die Oberfläche des Formkörpers hydrophil ausgebildet ist. Die Hydrophilierung der antimikrobiell ausgerüsteten Oberflächen bei oder oberhalb der Glastemperatur des antimikrobiellen Polymeren kann durch Kontakt mit Wasser oder Säuren, insbesondere verdünnten organischen oder Mineralsäuren, erfolgen. Die Anreicherung hydrohiler Gruppen, die oftmals Bestandteil antimikrobieller Polymere an der Grenzfläche des beschichteten Substrates sind, begünstigt die antimikrobielle Wirkung noch zusätzlich.

Der Formkörper kann eine keimabweisende und/oder kratzfeste Oberfläche aufweisen.

Schmutzabweisende Beschichtungen sind hoch abriebfest. Diese Antihaft-Beschichtungen eröffnen durch ihre hohe Abriebfestigkeit viele neue Anwendungsbereiche, bei denen nicht nur die Easy-to-clean-Eigenschaft, sondern auch eine hohe mechanische und chemische Beständigkeit gefordert ist. Die Schichten auf Basis der chemischen Nanotechnologie entstehen nicht mehr über den bislang genutzten Sol-Gel-Prozess, sondern durch organische Polymerchemie mit Integration keramischer Nanopartikel.

Kratzfeste Oberflächen kombinieren bspw. den Kunststoff Polyurethan mit Nanotechnologie. Damit lassen sich die überragenden Eigenschaften des vielseitig eingesetzten Materials, wie etwa Witterungsstabilität, Elastizität und Variationsbreite auf Kratzfestbeschichtungen mit integrierten keramischen Nanopartikeln übertragen. Die unsichtbaren Partikel werden an ihrer Oberfläche so modifiziert, dass sie sich in einem Rührprozess chemisch in das Polyurethan-Netzwerk einbinden lassen. Die transparenten Oberflächen eröffnen einer Reihe neuer Anwendungen für kratzfeste Oberflächen.

Der Formkörper kann eine keimabweisende selbstreinigende Oberfläche aufweisen.

Für selbstreinigende Oberflächen ist insbesondere ist daran gedacht, die Oberflächen leicht mit Wasser reinigen zu können. In welchem Grad ein Wassertropfen eine Oberfläche benetzt hängt vom Verhältnis zw. Energieverbrauch für eine Oberflächenvergrößerung und Energiegewinn durch Adsorption ab.

Auf rauen oder hydrophoben Oberflächen entsteht nur wenig Energie durch Adsorption, der Tropfen nimmt annähernd Kugelform an und kann "abrollen". Überrollt ein solcher Tropfen nun einen Schmutzpartikel auf einer rauen Oberfläche, so reduziert sich die Oberfläche des Tropfens relativ zur Luft, die Adsorptionsenergie nimmt zu, und der Partikel haftet an dem Tropfen und wird mitgezogen.

Auf einer glatten Oberfläche dagegen zerfließt der Wassertropfen partiell und kann den Partikel nicht mitnehmen, da dieser durch die Adhäsionskräfte zwischen Partikel und glatter Oberfläche stärker haftet, als auf einer rauen Oberfläche.

Es wird daher vorgeschlagen, die Oberflächen rau und hydrophob zu gestalten. Dabei kann es sich um eine Oberfläche mit einer künstlichen Oberflächenstruktur aus Erhebungen und Vertiefungen handeln, die selbstreinigende Eigenschaften aufweist. Bevorzugt sind die Oberflächenstrukturen im nm bis µm Maßstab. Besonders bevorzugt weisen die Oberflächenstrukturen einen im wesentlichen ähnlichen Abstand zueinander auf.

Ferner kann die Oberflächenstruktur zusätzlich Materialien mit antimikrobiellen Eigenschaften aufweisen. Außerdem kann die Oberfläche Partikel aufweisen, die mittels eines Trägersystems auf der Oberfläche fixiert sind.

Besonders bevorzugt ist, dass die Partikel aus einer Mischung von hydrophoben Partikeln und Partikeln mit antimikrobiellen Eigenschaften bestehen.

Dabei ist zumindest eine Oberfläche des Formkörpers aus einem Material, ausgewählt aus Polymeren, wie z. B. den Polyamiden, Polyurethanen, Polyetherblockamiden, Polyesteramiden, Polyvinylchlorid, Polyolefinen, Polysilikonen, Polysiloxanen, Polymethylmethacrylaten oder Polyterephthalaten sowie Metallen, Fasern, Geweben, Gläsern oder Keramiken gefertigt, dass selbstreinigende Eigenschaften aufweist.

Bei der Herstellung der Oberflächenstrukturen wird bevorzugt ein Material eingesetzt, welches antimikrobielle Eigenschaften aufweist. Dabei wird die Oberflächenstruktur, die Erhebungen oder Vertiefungen aufweist, vorzugsweise auf der Oberfläche selbst hergestellt.

Hierzu wird die Oberflächenstruktur durch Aufbringen und Fixieren von Partikeln auf der Oberfläche erzeugt. Zum Fixieren wird ein Trägersystem eingesetzt, wobei die Oberfläche, die Partikel und/oder das Trägersystem das antimikrobielle Material aufweisen können.

Als antimikrobielles Material wird ein Polymer eingesetzt, welches aus zumindest einem Monomeren ausgewählt aus Methacrylsäure-2-tert.-butylaminoethylester, Methacrylsäure-2-diethylaminoethylester, Methacrylsäure-2-diethylaminomethylester, Acrylsäure-2-tert.-butylaminoethylester, Acrylsäure-3-dimethylaminopropylester, Acrylsäure-2-diethylaminoethylester, Acrylsäure-2-dimethylaminoethyl-ester, Dimethylaminopropylmethacrylamid, Diethylaminopropylmethacrylamid, Acrylsäure-3-dimethylaminopropylamid, 2-Methacryloyloxyethyltrimethylammoniummethosulfat, Methacrylsäure-2-diethylaminoethylester, 2-Methacryloyloxyethyltrimethylammoniumchlorid, 3-Methacryloylaminopropyltrimethylammonium-chlorid, 2-Methacryloyloxyethyltrimethylammoniumchlorid, 2- Acryloyloxyethyl-4-benzoyldimethylammoniumbromid, 2-Methacryloyloxyethyl-4-benzoyldimethylammoniumbromid, 2-Acrylamido-2-methyl-1-propansulfonsäure, 2-Diethylaminoethylvinylether oder 3-Aminopropylvinylether hergestellt wurde.

Als Partikel wird eine Mischung aus Partikeln verwendet, die zumindest ein Material, ausgewählt aus Silikaten, dotierten Silikaten, Mineralien, Metalloxiden, Kieselsäuren oder Polymeren aufweisen, mit Homo- oder Copolymerpartikeln ausgewählt aus Methacrylsäure-2-tert.butylaminoethylester, Methacrylsäure-2-diethylaminoethylester, Methacrylsäure-2-diethylaminomethylester, Acrylsäure-2-tert.-butylaminoethylester, Acrylsäure-3-dimethylaminopropylester, Acrylsäure-2-diethylaminoethylester, Acrylsäure-2-dimethylaminoethylester, Dimethylaminopropylmethacrylamid, Diethylaminopropylmethacrylamid, Acrylsäure-3-dimethylaminopropylamid, 2-Methacryloyloxyethyltrimethylammoniummethosulfat, Methacrylsäure-2-diethylaminoethylester, 2-Methacryloyloxyethyltrimethylammoniumchlorid, 3-Methacryloylaminopropyltrimethylammonium-chlorid, 2-Methacryloyioxyethyltrimethylammoniumchlorid, 2- Acryloyloxyethyl-4-benzoyldimethylammoniumbromid, 2- Methacryloyloxyethyl-4-benzoyldimethylammoniumbromid, 2-Acrylamido-2-methyl-1-propansulfonsäure, 2-Diethylaminoethylvinylether oder 3-Aminopropylvinylether.

Dabei ist vorgesehen, dass die hydrophoben Partikel einen mittleren Partikeldurchmesser von 0,05 bis 30 nm aufweisen.

Hingegen weisen die Partikel mit antimikrobiellen Eigenschaften einen Durchmesser von 0,05 bis 2000 nm auf.

Diese Partikel bilden eine unregelmäßige Feinstruktur im Nanometerbereich auf der Oberfläche.

In einer bevorzugten Weiterbildung ist vorgesehen, dass der Formkörper eine keimabweisende strukturierte Oberfläche mit regelmäßigen und/oder unregelmäßigen Erhebungen und/oder Vertiefungen im nm und/oder µM Maßstab aufweist.

Vorzugsweise weisen die Oberflächen selbstreinigende- und/oder hydrophile- und/oder oleophobe- Eigenschaften und/oder eine niedrige Oberflächenenergie auf.

Dabei weist die Oberfläche zumindest eine fest verankerte Lage von Mikropartikeln auf, welche Erhebungen bilden. Die Erhebungen weisen eine mittlere Höhe von 20 nm bis 25 µm und einen mittleren Abstand von 20 nm bis 25 µm auf. Bevorzugt ist jedoch eine mittlere Höhe von 50 nm bis 4 µm und/oder ein mittlerer Abstand von 50 nm bis 4 µm.

Dabei sind die Mikropartikel nanostrukturierte Mikropartikel, die eine Feinstruktur mit Erhebungen bilden. Die Mikropartikel werden ausgewählt aus Partikeln von Silikaten, Mineralien, Metalloxiden, Metallpulvern, Kieselsäuren, Pigmenten, Metallen, Polymeren, pyrogenen Kieselsäuren, Fällungskieselsäuren, Aluminiumoxid, Mischoxiden, dotierten Silikaten, Titandioxiden oder pulverförmigen Polymeren und die bevorzugt hydrophobe Eigenschaften aufweisen.

Dabei ist das Flächenextrudat selbst ein Material, ausgewählt aus Polycarbonaten, Polyoxymethylenen, Poly (meth) acrylaten, Polyamiden, Polyvinylchlorid, Polyethylenen, Polypropylenen, aliphatischen linearen- oder verzweigten Polyalkenen, cyclischen Polyalkenen, Polystyrolen, Polyestern, Polyacrylnitril oder Polyalkylenterephthalaten, Poly (vinylidenfluorid), oder andere Polymere aus Poly (isobuten), Poly (4-methyl-1- penten),Polynorbornen als Homo-oder Copolymer, ein Polymer auf der Basis von Polycarbonaten, Polyoxymethylenen, Poly (meth) acrylaten, Polyamiden, Polyvinylchlorid, Polyethylenen, Polypropylenen, aliphatischen linearen- oder verzweigten Polyalkenen, cyclischen, Polyalkenen, Polystyrolen, Polyestern, Polyacrylnitril oder Polyalkylenterephthalaten, Poly-(vinylidenfluorid), oder andere Polymere aus Poly (isobuten), Poly (4-methyl-1-penten), Polynorbonen als Homo-oder Copolymer sowie deren Gemische sowie deren Gemische, aufweist.

Ferner weist es selbstreinigende Eigenschaften und durch Mikropartikel gebildete Erhebungen auf, wobei zur Herstellung in die Oberfläche des Flächenextrudates hydrophobe Mikropartikel eingedrückt werden. Die eingesetzten Mikropartikel weisen einen mittleren Partikeldurchmesser von 0,02 bis 100 µm auf.

Die strukturierten Oberflächen können eine niedrige Oberflächenenergie, aufweisen.

Die strukturierten Oberflächen können Erhebungen mit einer mittleren Höhe von 10 nm bis 200 µm und einem mittleren Abstand von 10 nm bis 200 µm aufweisen, und deren äußere Form kann durch eine mathematische Kurven und/oder Funktionen mit einer Symmetrie bezüglich einer Ebene beschrieben sein.

Geeignete Materialien sind z. B. Gold, Titan, Silizium, Kohlenstoff, Quarzglas, Lithiumniobat, Siliciumnitrid, Hydroxylapatit, PMMA, Silikone, Epoxydharze, Polydioxanon, Polyamid, Polyimid. Die Charakterisierung von Oberflächen bezüglich ihrer Benetzbarkeit kann über die Messung der Oberflächenenergie erfolgen. Diese Größe ist z. B. über die Messung der Randwinkel am glatten Material von verschiedenen Flüssigkeiten zugänglich (D. K. Owens, R. C. Wendt, J. Appl. Polym. Sci. 13, 1741 (1969)) und wird in mN/m (Milli-Newton pro Meter) angegeben. Nach Owens et al. bestimmt, weisen glatte Polytetrafluorethylen-Oberflächen eine Oberflächenenergie von 19,1 mN/m auf.

Eine besonders niedrige Oberflächenenergie ist insbesondere dann notwendig, wenn nicht nur hydrophobes, sondern auch oleophobes Verhalten gefordert ist. Dies ist insbesondere bei nichtfesten, öligen Verschmutzungen der Fall.
Zur Herstellung wird die strukturierte, hydrophobe Oberfläche mit Erhebungen und Vertiefungen mit einem Additiv versetzt, dass eine Partikelgröße von 0,0001 bis 20 µm aufweist und einer organischen Matrix, welche zumindest einen thermoplastischen, elastomeren oder duroplastischen Kunststoff aufweist.

Diese Oberfläche weist selbstreinigende Eigenschaften auf, ist kratzfest, abriebfest und/oder anfärbbar. Ferner ist die Glas- oder Kunststoffoberfläche entspiegelt.

Insbesondere wenn die Oberfläche mit hydrophoben Eigenschaften ausgestattet ist, ist sie schwer von Wasser oder wässrigen Lösungen benetzbar und weist damit selbstreinigende Eigenschaften auf, da Verunreinigungen durch bewegtes Wasser entfernt werden können.

Eine Vorrichtung mit Oberflächen, die flüssigkeitsabweisende Eigenschaften und Oberflächenstrukturen mit Erhebungen aufweisen, zeichnet sich dadurch aus, dass die Oberflächen vorzugsweise Kunststoffoberflächen sind, in die Mikropartikel direkt eingebunden und nicht über Trägersysteme oder ähnliches angebunden sind.

Die Vorrichtungen selbst können als Material vorzugsweise Polymere auf der Basis von Polycarbonaten, Polyoxymethylenen, Poly(meth)acrylaten, Polyamiden, Polyvinylchlorid (PVC), Polyethylenen, Polypropylenen, Polystyrolen, Polyestern, Polyethersulfonen, aliphatischen linearen- oder verzweigten Polyalkenen, cyclischen Polyalkenen, Polyacrylnitril oder Polyalkylenterephthalaten sowie deren Gemische oder Copolymere, auf. Besonders bevorzugt weisen die Spritzgusskörper als Material ein Material, ausgewählt aus Poly(vinylidenfluorid), Poly(hexafluorpropylen), Poly(perfluorpropylenoxid), Poly(fluoralkylacrylat), Poly(fluoralkylmethacrylat), Poly(vinylperfluoralkylether) oder andere Polymere aus Perfluoralkoxyverbindungen, Poly(ethylen), Poly(propylen), Poly(isobuten), Poly(4-methyl-1-penten) oder Polynorbonen als Homo- oder Copolymer aufweisen. Ganz besonders bevorzugt weisen die Spritzgusskörper als Material ihr die Oberfläche Poly(ethylen), Poly(propylen), Polymethylmethacrylaten, Polystyrolen, Polyestern, Acrylnitril- Butadien-Styrol Terpolymere (ABS) oder Poly(vinylidenfluorid) auf.

Mikropartikel, die die Erhebungen auf der Oberfläche der Vorrichtung bilden, sind vorzugsweise ausgewählt aus Silikaten, Mineralien, Metalloxiden, Metallpulvern, Kieselsäuren, Pigmenten oder Polymeren, ganz besonders bevorzugt aus pyrogenen Kieselsäuren, Fällungskieselsäuren, Aluminiumoxid, Siliziumoxid, dotierten Silikaten, pyrogenen Silikaten oder pulverförmige Polymeren.

Bevorzugte Mikropartikel weisen eine unregelmäßige Feinstruktur im Nanometerbereich an der Oberfläche auf, einen Partikeldurchmesser von 0,02 bis 100µm, besonders bevorzugt von 0,1 bis 50 µm und ganz besonders bevorzugt von 0,1 bis 10µm auf. Geeignete Mikropartikel können aber auch einen Durchmesser von kleiner als 500 nm aufweisen oder sich aus Primärteilchen zu Agglomeraten oder Aggregaten mit einer Größe von 0,2 bis 100 µm zusammenlagern.

Bevorzugte Mikropartikel sind solche Partikel, die zumindest eine Verbindung, ausgewählt aus pyrogener Kieselsäure, Fällungskieselsäuren, Aluminumoxid, Siliziumdioxid, pyrogenen und/oder dotierten Silikaten oder pulverförmige Polymeren aufweisen.

Es kann vorteilhaft sein, wenn die Mikropartikel hydrophobe Eigenschaften aufweisen, wobei die hydrophoben Eigenschaften auf die Materialeigenschaften der an den Oberflächen der Partikel vorhandenen Materialien selbst zurückgehen können oder aber durch eine Behandlung der Partikel mit einer geeigneten Verbindung erhalten werden können. Die Mikropartikel können vor oder nach dem Aufbringen bzw. Anbinden auf bzw. an die Oberfläche der Vorrichtung bzw. des Spritzgusskörpers mit hydrophoben Eigenschaften ausgestattet worden sein.

Vorzugsweise sind die Oberflächen mit flüssigkeitsabweisenden Eigenschaften hydrophob, wobei das unstrukturierte Material eine Oberflächenenergie weniger als 35 mN/m, bevorzugt von 10 bis 20 mN/m aufweist.

Es wird ferner ein Verfahren zur Herstellung von Kunststoff-Granulaten und -Pulvern, beschrieben.

Eine Kombination aus Mikro- und Nanostrukturierung kann einfach und leicht genutzt werden, um die Benetzung von Oberflächen zu verändern.

Das Aufbringen des die Partikel aufweisenden Lösemittels auf die Polymeroberfläche kann z.B. durch Aufsprühen, Aufrakeln, Auftropfen oder durch Eintauchen der Polymeroberfläche in das die Partikel aufweisende Lösemittel erfolgen.

Durch das Verfahren können selbstreinigende Polymeroberfläche hergestellt werden, die eine künstliche, zumindest teilweise hydrophobe Oberflächenstruktur aus Erhebungen und Vertiefungen aufweist, die sich dadurch auszeichnen, dass die Erhebungen und Vertiefungen durch an der Polymeroberfläche fixierte Partikel gebildet werden.

Die Partikel können auch als Aggregate oder Agglomerate vorliegen, wobei gemäß DIN 53 206 unter Aggregaten flächig oder kantenförmig aneinander gelagerte Primärteilchen (Partikel) und unter Agglomeraten punktförmig aneinander gelagerte Primärteilchen (Partikel) verstanden werden.

Die beschriebenen Strukturen können z. B. durch ein Spritzgussverfahren in Kombination mit einem durch LIGA-Verfahren hergestellten, konventionellen Spritzgusswerkzeug hergestellt werden. Mit diesem Werkzeug werden Strukturen in Poly(propylen) abgeformt. Anschließend wurde die Form einer UV-Strahlung von 254 nm für zwei Minuten ausgesetzt. Auf die so aktivierten Oberflächen wurde thermisch Fluoralkylacrylat aufgepfropft Durch diese Vorgehensweise wurde die Oberflächenenergie von etwa 28 mN/m auf weniger als 15 mN/m reduziert.

Hierbei ist es nicht nötig, antimikrobielle Polymere in das Substrat mit einzuarbeiten, wie das z. B. bei der Compoundierung von Formmassen geschieht. Ein weiterer Vorteil des Verfahrens ist die ökonomisch effiziente Einsparung von antimikrobiellen Polymeren, die bei einer Compoundierung z. B. wirkungslos in der Matrix der Formmasse verbleiben. Weiterhin kann man auf diese Weise unerwünschte Veränderungen der physikalischen Eigenschaften der Substrate weitgehend ausschließen, da nur eine sehr dünne Schicht an der Oberfläche des Substrates verändert wird. Das erfindungsgemäße Verfahren kann mit anderen Methoden zur Oberflächennachbehandlung problemlos kombiniert werden. So ist z. B. möglich, nach der thermisch unterstützten Auftragung der Polymere eine Hydrophilierung mit Wasser bzw. Säuren durchzuführen.
Die Werkstoffeigenschaften der verschiedenen Kunststoffe werden nur unwesentlich beeinflusst. Kennwerte wie Dauergebrauchstemperatur, Kriechfestigkeit, thermische und elektrische Isolation bleiben erhalten. Das Compound ist unter allen erdenklichen Herstellungs-, Verarbeitungs- und Anwendungsbedingungen einsetzbar. Es kommt bei Halbzeugen aus PEEK, PPSU, POM-C, PET sowie bei Spritzgussteilen, extrudierten Profilen und kalandrierten Platten zum Einsatz.

Zur Herstellung von erfindungsgemäßen Formkörpern können alternativ und/oder ergänzend zu den vorgenannten Verfahren die folgenden Verfahren angewandt werden.
- Plasmaverfahren
- Laserverfahren
- Sol-Gel-Verfahren
- Galvanische Verfahren
- Erzeugung von Nanostrukturen durch Selbstorganisation
- Nanostrukturierung von Materialien und Oberflächen
- Aufdampfen im Hochvakuum (Elektronenstrahlverdampfen)
- Aufdampfen im Hochvakuum (Widerstandstiegelverdampfen)
- Kathodenzerstäubung
- CVD-Verfahren (Chemical Vapour Deposition)
- PVD (Physical Vapour Deposition)
- LIGA-Verfahren
- Thermische Oxidation
- Mikrogalvanik (Hartlegierungsabscheidungen)
- Spritzguß von Mikrokomponenten aus Kunststoffen
- Vakuumbeschichtung
- chemisch galvanischen Beschichtung
- lnmould-coating
- Pre-coating

Außerdem gehört zur Erfindung ein Verfahren zur Herstellung von erfindungsgemäßen Formkörpern. Vorteilhafte Ausgestaltungen des erfindungsgemäßen Verfahrens sind in den Unteransprüchen angegeben.

In den Zeichnungen sind Ausführungsbeispiele der Erfindung schematisch dargestellt. Es zeigen:
- Fig. 1: ein medizintechnisches Gerät,
- Fig. 2: ein Gehäuseteil eines medizintechnischen Gerätes,
- Fig. 3: ebenfalls ein Gehäuseteil eines medizintechnische Gerätes,
- Fig. 4: ein Oberflächenprofil,
- Fig. 5 - 10: verschiedene Oberflächentopographien.
- Fig. 11: eine perspektivische Darstellung eines Befeuchters, der zwischen das Beatmungsgerät und einen Beatmungsschlauch einsetzbar ist,
- Fig. 12: ein Beatmungsgerät mit Zuschaltventil zur Bereitstellung einer erhöhten Sauerstoffkonzentration,
- Fig. 13: eine perspektivische Darstellung einer Beatmungsmaske mit Stirnstütze und
- Fig. 14: eine vergrößerte teilweise Querschnittdarstellung von zwei zusammengefügten Bauelementen.

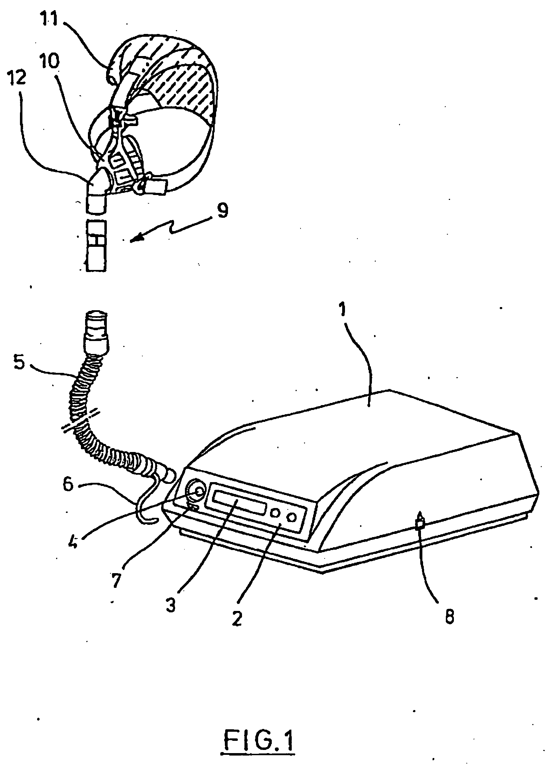

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird eine als Verbindungsschlauch ausgebildete Verbindungsleitung (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung der Verbindungsleitung (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Jedes der o.g. Funktionselemente ist mit jeweils für den Einsatzzweck optimierten modifizierten Kunststoffen ausgestattet, um bestmöglich geeignet zu sein. Die Maske (10) ist beispielsweise mit einer bioziden und einer nichtbeschlagenden Oberfläche versehen. Das Beatmungsgerät (1) ist aus als Gehäuseteile ausgebildeten Formkörpern gebildet, die an den Oberflächen mit einer kratzfesten, bioziden, selbstreinigenden und fotokatalytischen Oberfläche ausgestattet sind. Die Innenflächen des Beatmungsgerätes (1), insbesondere der Schalldämpfer (nicht dargestellt) ist biozid und/oder selbstreinigend. Der Eingangsfilter ist fotokatalytisch und biozid.

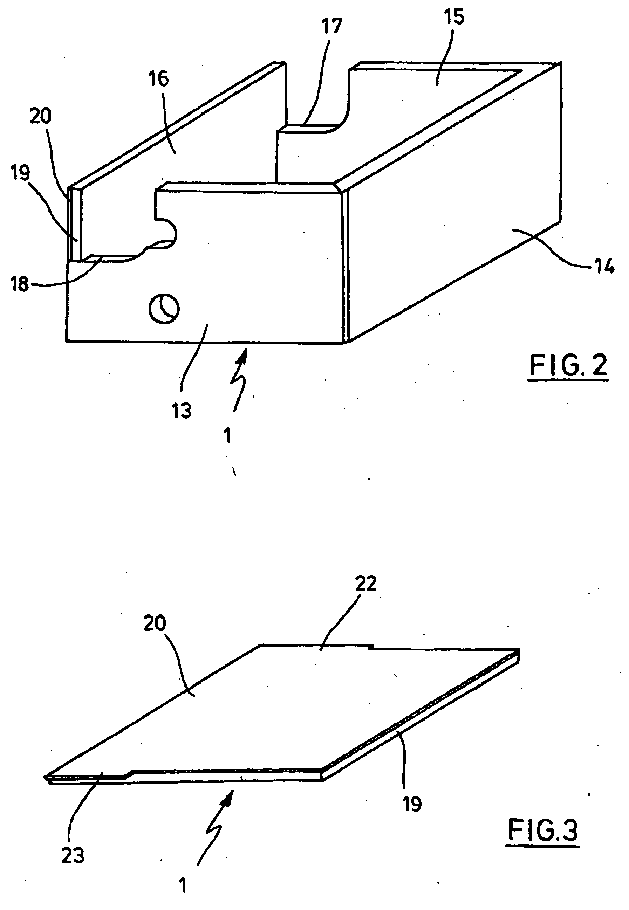

Fig. 2 zeigt ein weiteres Gerätegehäuse (1), das aus plattenförmigen Wandungen (13, 14, 15, 16) besteht. Das Gerätegehäuse (1) ist mit einer Eingangsausnehmung (17) sowie einer Ausgangsausnehmung (18) versehen. Die Wandungen (13, 14, 15, 16) bestehen aus einem Grundmaterial (19), das mit einer Oberflächenbeschichtung (20) versehen ist. Das Gerätegehäuse (1) gemäß Fig. 2 kann beispielsweise als eine Schalldämmbox ausgebildet sein, die zur Aufnahme eines Gebläses oder eines Kompressors vorgesehen ist.
Fig. 3 zeigt ein Deckelteil (21) für das Gerätegehäuse (1) gemäß Fig. 2. Auch das Deckelteil (21) besteht aus einem Grundmaterial (19) und einer Oberflächenbeschichtung (20). Im Bereich der Ausnehmungen (17, 18) des Gerätegehäuses (1) kann das Deckelteil (21) Vorsprünge (22, 23) aufweisen.
Die Oberflächenbeschichtungen (20) können in unterschiedlichen Verfahren hergestellt werden, die teilweise vorstehend bereits beispielhaft erläutert worden. Die Oberflächenbeschichtungen können durch die erläuterten Einbringungen von Partikeln erzeugt werden, es sind aber auch Bedampfungen, Kaschierungen oder Plasmabeschichtungen verwendbar. Ebenfalls können die bereits erläuterten Verfahren zum Aufbringen von flüssigen Beschichtungen in Reinform oder verdünnt mit Lösungsmitteln zum Einsatz kommen. Auch Oberflächenbearbeitungen, beispielsweise unter Verwendung mechanischer Mittel, Laser- oder Elektronenstrahlen sind möglich.

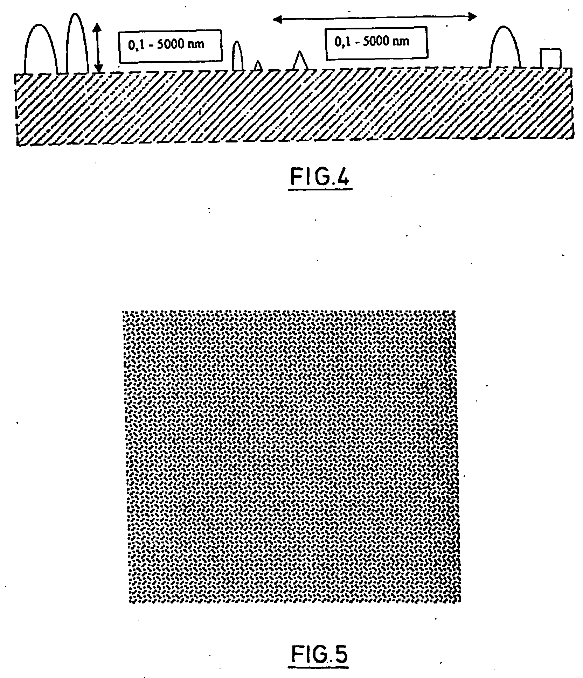

Fig. 4 zeigt einen Abschnitt eines Oberflächenprofils eines modifizierten Formkörpers für ein medizintechnisches Gerät mit Erhebungen verschiedener Form, die eine Höhe bezogen auf den Untergrund von 0,1 nm bis 5000 nm aufweisen. Der Abstand von den einzelnen Erhebungen liegt ebenfalls im Bereich von 0,1 nm bis 5000 nm.
Diese Erhebungen sind in verschiedenen Formen regelmäßige Strukturen bildend auf der Oberfläche angeordnet.

Ähnlich zur Erfindung können beispielsweise folgende Zubehörteile im Bereich der Beatmung eingesetzt werden.

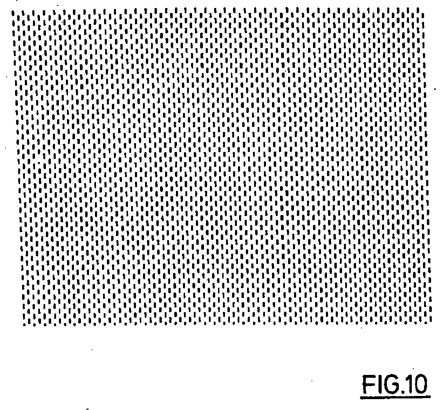

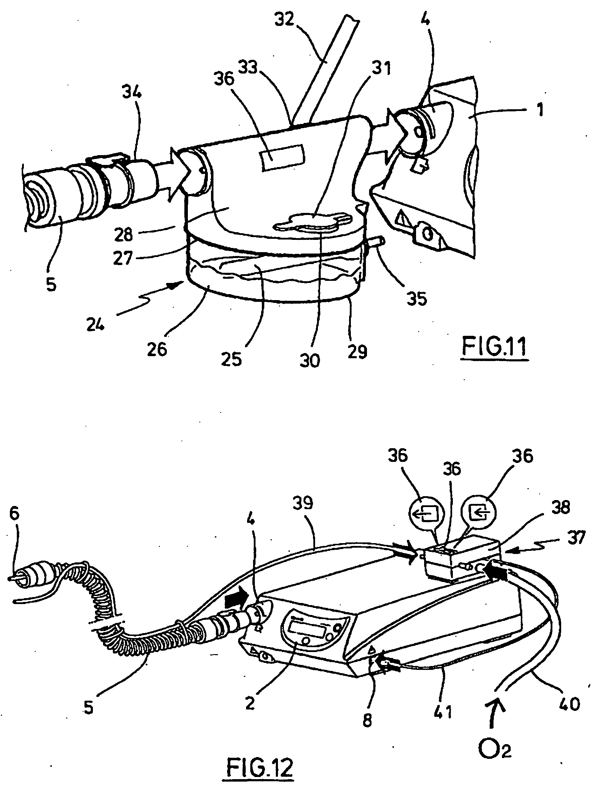

Anfeuchter (Fig. 10), 02-Ventil (Fig. 12), Kopfhaube, Patienten Interface (beispielsweise Maske, Nasal pillows, Tubus) Schlauch, Filter, Halterung, Kupplung, Heizung, Wechselteile, Tasche. Im folgenden wird erläutert, wie die Erfindung zur Verbesserung der genannten Zubehörteile beiträgt.

Eine weitere Variante besteht darin, daß die Verminderung eines Zeitaufwandes für eine erforderliche Reinigung wenigstens 10 % beträgt.

Im Hinblick auf auftretende Oberflächenreflexionen kann die funktionale Eigenschaft darin bestehen, daß zumindest für bestimmte Wellenlängen und/oder bestimmte Einfallswinkel eine die Reflektion senkende der die Oberfläche entspiegelnde Wirkung hervorgerufen wird.

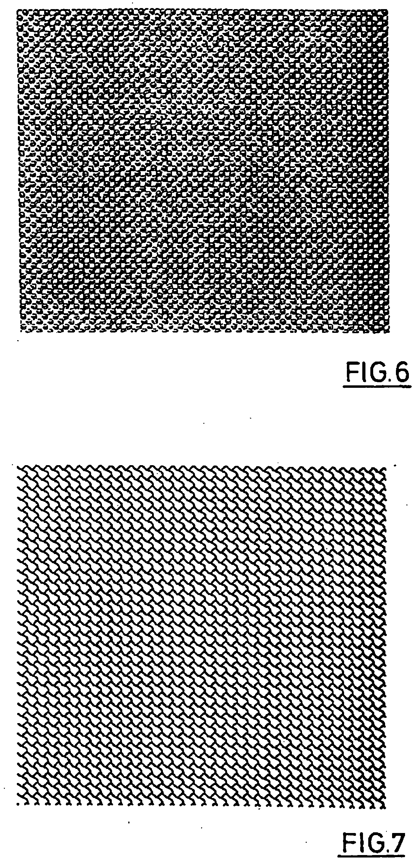

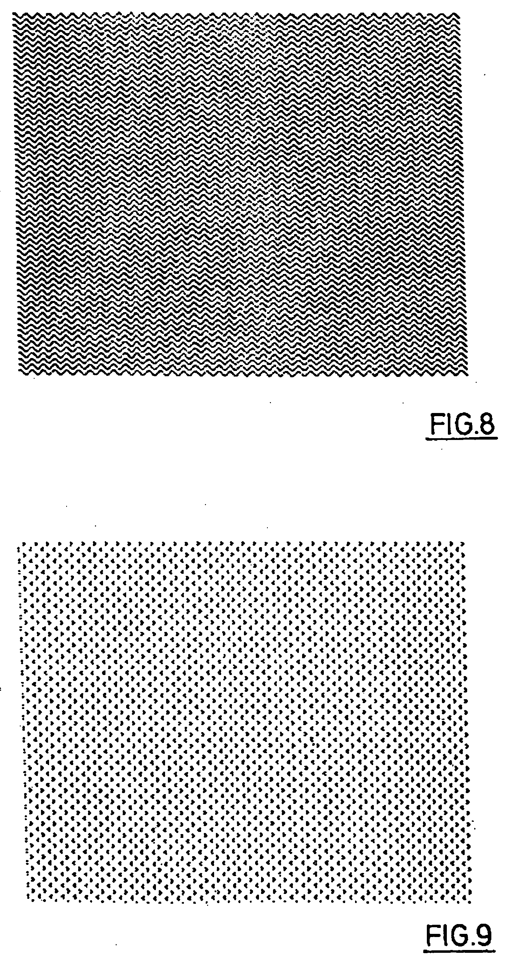

Es wird daher vorgeschlagen die Reibungskraft der Oberflächen herabzusetzen, um Energie einzusparen und/oder die Geräuschentwicklung zu begrenzen. Die erfindungsgemäßen widerstandsverminderten Oberflächen (Beispiele siehe Fig. 4 - 9) bestehen aus mikroskopisch kleinen Strukturen der Oberfläche beispielsweise Rillen, die bevorzugt parallel zur Strömung des Mediums ausgerichtet sind. Bekannt sind solche Oberflächen aus der Natur, beispielsweise von der Haihaut. Die Oberflächenstrukturen sind so dimensioniert sein, daß sie für das strömende Medium wie eine hydraulisch glatte Oberflächen wirken. Die widerstandsverminderte Wirkung besteht in einer Behinderung der turbulenten Anteile der Strömung an der Wand.

Bevorzugt weisen die Oberflächenstrukturen einen im wesentlichen gleichen Abstand zueinander auf, der im Bereich 100 nm - 200 µm liegt. Besonders bevorzugt im Bereich 5 µm bis 100 µm. Besonders bevorzugt weisen die erfindungsgemäßen Oberflächen eine Widerstandsverminderung im Bereich von > 1,0 % auf.
Der luftführende Teil eines Beatmungsgerätes und/oder Schlauches weist erfindungsgemäß wenigstens abschnittsweise eine strukturierte Oberfläche mit regelmäßigen und/oder unregelmäßigen Erhebungen auf und/oder weist eine Oberfläche auf, die die Reibung eines strömenden Mediums vermindert und /oder weist eine strömungsoptimierte Oberfläche auf.

Zur Vermeidung eines Austrocknens der Atemwege wird typischerweise eine Befeuchtung der Atemluft durchgeführt. Da Patienten erwärmte Luft als angenehm empfinden und da durch eine Beheizung beispielsweise mittels eines Heizelements (25) die Luft mehr Wasserdampf aufnehmen kann, erfolgt typischerweise eine Beheizung eines als Flüssigkeitsreservoir (26) verwendeten Wasservorrats des Befeuchtungssystems indirekt und/oder direkt beispielsweise über den metallischen Boden des Wasservorrats beziehungsweise beispielsweise mittels eines Heizstabes (25) direkt im Wasser. Ein Atemgasbefeuchter kann über eine Kopplung (4) extern außenseitig an ein Beatmungsgerät angeschlossen sein und/oder innerhalb eines Beatmungsgerätes angeordnet sein. Aufgrund der einzuhaltenden hygienischen Anforderungen muß der Anfeuchter zum Reinigen demontierbar sein und trotzdem eine ausreichende Abdichtung gegen das Wasser gewährleisten. Der Anfeuchter gliedert sich in ein, im wesentlichen luftführendes, Oberteil (28), welches auch der Verbindung von Beatmungsgerät (1) und Verbindungsschlauch (5) dient, und eine Unterteil (29) das den Wasservorrat aufnehmen kann. Im Bereich des Oberteiles (28) ist eine Einfüllöffnung für Flüssigkeit (30) mit Verschluß (31) angeordnet.

Im Bereich des Anfeuchters kann eine Gasleitung (32) angeordnet sein, die bevorzugt als Druckmeßleitung und/oder Sauerstoffzuleitung ausgebildet ist. Die Gasleitung ist über eine Gaskupplung (33) mit dem Anfeuchter verbunden. Der Anfeuchter ist über einen Anschlußadapter (34) mit einem Verbindungsschlauch (5) koppelbar. Eine Kommunikation mit dem Beatmungsgerät (1) kann über eine vorhandene Steckverbindung (35) zwischen dem Anfeuchter und dem Beatmungsgerät realisiert werden. Im Bereich des Anfeuchters kann eine Anzeige (36) positioniert werden.

Üblicherweise wird eine Dichtung (27) zwischen zwei demontierbaren Teilen des Anfeuchters angeordnet, um den Austritt von Wasser und/oder Atemgas zu verhindern. Insbesondere muß der Anfeuchter von innen regelmäßig gereinigt werden. Typischerweise werden Anfeuchter nach dem Stand der Technik in einer Spülmaschine gereinigt und gegebenenfalls zusätzlich desinfiziert, der Aufwand für die Pflege und die Reinigung ist somit hoch.

Dieser Nachteil wird dadurch behoben, daß der Anfeuchter demontierbar ist und daß die Oberflächen des Anfeuchters wenigstens abschnittsweise die funktionalen Oberflächen aufweisen.

Oberflächen sind Flächen des Anfeuchters, die mit einem Medium wie Luft oder Wasser in Kontakt stehen. Die Oberflächen können hierbei innere Oberflächen des Anfeuchters sein, wie beispielsweise die Innenseite des Wasservorrats und/oder die Innenseite des luftführenden Teils des Anfeuchters, es ist jedoch ebenso die nach außen weisende Oberfläche gemeint.

Kontaktstellen sind Flächen des Anfeuchters und/oder Beatmungsgerätes und/oder Verbindungsschlauches und/oder des Patienteninterface und/oder des Sauerstoffzuschaltventils, die mit einem anderen Formkörper in Kontakt stehen. Die Kontaktstellen können hierbei innere Kontaktstellen eines Formkörpers sein, wie beispielsweise die Kontaktstelle zwischen Unterteil (29) und Oberteiles (28) des Anfeuchters, es ist jedoch ebenso die nach außen weisende Kontaktstelle gemeint wie beispielsweise die Kontaktstelle zwischen Anfeuchter und Beatmungsgerät.

Ein weiteres Anwendungsbeispiel wird nachfolgend in Bezug auf ein Sauerstoffzuschaltventil (37) erläutert. Ein Sauerstoffzuschaltventil (37) ist eine Einrichtung zur Zufuhr von Sauerstoff zu einem Anwender.

Fig. 12 zeigt eine perspektivische Darstellung eines Beatmungsgerätes (1), das mit einer Kopplung (4), einem Bedienfeld (2) versehen ist. Mit der Kopplung (4) ist ein Verbindungsschlauch (5) verbunden, durch den sich eine Druckmeßleitung (6) hindurch erstreckt.

Außenseitig am Verbindungsschlauch (5) ist eine Sauerstoffleitung (39) verlegt, die mit einem Sauerstoff-Zuschaltventil (37) verbunden ist. Das Zuschaltventil (39) ist über eine Versorgungsleitung (40) an eine nicht dargestellte Sauerstoffquelle angeschlossen. Über eine Steuerleitung (41) ist das zuschaltventil (39) mit einer Schnittstelle (8) des Beatmungsgerätes (1) verbunden.

Bei dem in Fig. 12 dargestellten Beispiel ist das Zuschaltventil (39) außenseitig am Beatmungsgerät (1) angeordnet. Es ist jedoch ebenso daran gedacht, das Zuschaltventil in das Beatmungsgerät zu integrieren.

Bevorzugt weist ein Sauerstoffzuschaltventil (37) wenigstens abschnittsweise keimabweisend ausgebildete Oberfläche auf. Die Oberflächen (38) des Sauerstoffzuschaltventils sind beispielsweise wenigstens abschnittsweise durch Verringerung der Oberflächenadhäsion keimabweisend ausgebildet. Die Oberflächen des Sauerstoffzuschaltventils sind beispielsweise wenigstens abschnittsweise durch Abtötung und/oder Wachstumshemmung von Mikroorganismen keimabweisend ausgebildet. Beispielsweise weisen die Oberfläche des Sauerstoffzuschaltventils Biozide auf. Die Oberfläche des Sauerstoffzuschaltventils weist wenigstens abschnittsweise bevorzugt als Wirkstoff Silber und/oder silberhaltige Verbindungen auf. In einem anderen Ausführungsbeispiel weist die Oberfläche als Wirkstoff ein antimikrobiell wirksames Polymer auf.

Ein weiteres Anwendungsbeispiel wird in Bezug auf eine Kopfhaube nachfolgend erläutert. Wie Fig. 1 zeigt, ist eine Beatmungsmaske (10) typischerweise mit einer Kopfhaube (11) im Bereich des Kopfes fixiert. Die Kopfhaube weist üblicherweise mindestens ein Band auf, daß im Bereich seiner der Beatmungsmaske abgewandten Ausdehnungen miteinander verbunden ist und maskenseitig an zumindest zwei Stellen mit der Maske kontaktiert. Die Befestigung der Kopfhaube an der Maske erfolgt üblicherweise über Kontaktmechanismen wie beispielsweise Klips und/oder Haken und/oder Schlaufen. Die Kopfhaube kann zusätzlich durch ein Kinnband ergänzt werden.

Bedingt durch den ständigen Kontakt zum Patienten muß die Kopfhaube gereinigt werden können. Bei Kopfhauben nach dem Stand der Technik, die Typischerweise in der Waschmaschine gewaschen werden, ist die einwandfreie hygienische Aufbereitung nicht sichergestellt. Dieser Nachteil wird erfindungsgemäß dadurch gelöst, daß die Oberflächen der Kopfhaube und/oder die Oberflächen der Kontaktmechanismen wie beispielsweise Klips und/oder Haken und/oder Schlaufen wenigstens abschnittsweise die erfindungsgemäßen funktionalen Oberflächen aufweisen. Im folgenden Oberflächen der Kopfhaube genannt. Oberflächen der Kopfhaube können auch innere Oberflächen sein, wenn beispielsweise ein grobporiges Gewebe verwandt wird.

Bevorzugt weist die Kopfhaube wenigstens abschnittsweise keimabweisend ausgebildete Oberfläche auf. Die Oberflächen der Kopfhaube sind beispielsweise wenigstens abschnittsweise durch Verringerung der Oberflächenadhäsion keimabweisend ausgebildet. Die Oberflächen der Kopfhaube sind beispielsweise wenigstens abschnittsweise durch Abtötung und/oder Wachstumshemmung von Mikroorganismen keimabweisend ausgebildet. Beispielsweise weisen die Oberfläche der Kopfhaube Biozide auf. Die Oberfläche der Kopfhaube weist wenigstens abschnittsweise bevorzugt als Wirkstoff Silber und/oder silberhaltige Verbindungen auf. In einem anderen Ausführungsbeispiel weist die Oberfläche als Wirkstoff ein antimikrobiell wirksames Polymer auf.

Die Kopfhaube kann bevorzugt eine keimabweisende und/oder selbstreinigende und/oder hydrophobe und/oder oleophobe und/oder fotokatalytische und/oder elektrisch leitfähige Oberfläche besitzen.

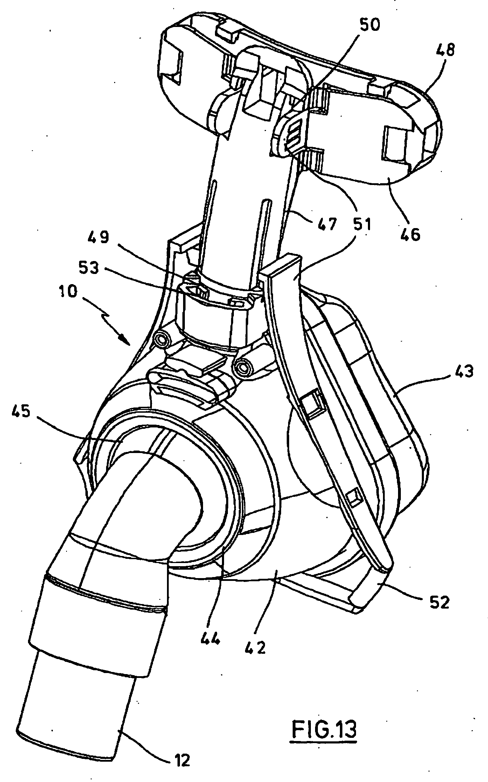

Als nächstes Anwendungsbeispiel wird nachfolgend ein Patienteninterface erläutert. Gemäß dem in Fig. 13 dargestellten Ausführungsbeispiel ist ein Patienteninterface als eine Gesichts-Maske (10) ausgeführt. Üblicherweise ist eine Maske als ein modulartiges System ausgeführt und besteht typisch aus folgenden Bauteilen, wobei diese Aufzählung nicht erschöpfend ist: Maskenkörper (42) und/oder Maskenwulst (43) und/oder Ausatemsystem (44) und/oder Kupplungselement (12) und/oder Gelenk (45) und/oder Stirnstütze (46) und/oder Halterung der Stirnstütze (47) und/oder Stirnpolster (48) und/oder Befestigungsvorrichtung (52) für eine Kopfbänderung Sicherungsring und/oder Reißleine. Nicht alle einzelnen Bauteile müssen zwingend im Bereich einer Maske angeordnet sein, damit diese funktionsfähig ist.

Der Maskenwulst (43) dient zur Anlage am Gesicht eines Patienten und gewährleistet die erforderliche Abdichtung. Über ein Gelenk ist der Maskenkörper mit einem Kupplungselement (12) verbunden, die zum Anschluß an einen Atemgasschlauch dient. Zur Gewährleistung einer sicheren Positionierung der Atemmaske im Kopfbereich eines Patienten wird eine Stirnstütze (47) verwendet, die mit einem Stirnpolster (48) ausgestattet ist. Die Stirnstütze ist über eine Halterung (53) mit dem Maskenkörper verbunden. Eine Grob (49) - und Fein (50) - Verstellung für die Stirnstütze dient der Justierung der Masken am Patienten. Die Feinverstellung kann beispielsweise über feine Rastungen an der Stirnstütze bzw. am Stirnpolster erfolgten. Die Grobverstellung erfolgt beispielsweise durch ein Umstecken der Stirnstütze im Bereich der Halterung (53). Mit Hilfe der Grobverstellung rückt der Patient beispielsweise die Stirnstütze in die gewünschte Position und fixiert die Stirnstütze unter Verwendung der Feinverstellung am Stirnpolster in der ausgewählten Position.

Für die Justierung einer Masken finden komplexe Schwenk- oder Schiebemechanismen ebenfalls Verwendung. Die Verstellmechanismen weisen jeweils Bedienflächen (51) für den Anwender auf. Durch eine leichte mechanische Einwirkung eines Anwenders im Bereich der Bedienflächen lassen sich die Masken-Bauteile relativ zueinander bewegen. Wenn die Beatmungsmaske ohne Stirnstütze verwendet wird, kann in die Steckverbindung ein Blindstopfen eingesetzt werden oder es ist die Verwendung eines Adapters möglich, der eine Kopplung mit der Kopfbänderung ermöglicht. Das Stirnpolster kann bevorzugt von der Stirnstütze lösbar sein. Hierdurch kann eine leichte Austauschbarkeit und/oder getrennte Reinigung erfolgen.
Der Maskenwulst ist Typischerweise relativ zum Maskenkörper abnehmbar. Die Fixierung des Maskenwulstes im Maskenkörper erfolgt beispielsweise durch Einschnitte, Stege, Verdickungen oder Aussparungen.

Im Bereich des Maskenkörpers kann ein Löseelement angebracht werden, die es dem Patienten ermöglicht, mit einem einzigen Zug eine Verbindung zwischen dem Beatmungsschlauch und der Atemgasmaske zu lösen.
Alternativ zu einer Maske werden verschiedene andere Patienten-Interfaces verwandt. Beispielhaft sind folgende genannt: Nasenstopfen (Nasal pillows), Tuben, Tracheostoma, Katheter.

Eine wichtige Voraussetzung für Patienten-Interfaces ist, daß Sie einfach und effektiv zu reinigen sein müssen. Hierzu finden die erfindungsgemäßen Oberflächen im Bereich der Masken Verwendung. Im folgenden werden Masken und alle Masken-Bauteile, sowie andere Patienten-Interfaces wie Nasal pillows vereinfacht unter dem Begriff Patienten-Interfaces zusammengefasst.

Bevorzugt weisen Patienten-Interfaces wenigstens abschnittsweise keimabweisend ausgebildete Oberfläche auf. Die Oberflächen der Patienten-Interfaces sind beispielsweise wenigstens abschnittsweise durch Verringerung der Oberflächenadhäsion keimabweisend ausgebildet. Die Oberflächen der Patienten-Interfaces sind beispielsweise wenigstens abschnittsweise durch Abtötung und/oder Wachstumshemmung von Mikroorganismen keimabweisend ausgebildet. Beispielsweise weisen die Oberflächen der Patienten-Interfaces Biozide auf. Die Oberflächen der Patienten-Interfaces weisen wenigstens abschnittsweise bevorzugt als Wirkstoff Silber und/oder silberhaltige Verbindungen auf. In einem anderen Ausführungsbeispiel weisen die Oberfläche als Wirkstoff ein antimikrobiell wirksames Polymer auf. Besonders bevorzugt sind die Bedienflächen biozid und/oder keimabweisend.

Patienten-Interfaces können bevorzugt wenigstens abschnittsweise eine keimabweisende und/oder selbstreinigende und/oder hydrophobe und/oder oleophobe und/oder fotokatalytische und/oder kratzfeste und/oder beschlagfreie und/oder hautfreundliche und/oder reibungsarme und/oder elektrisch leitfähige Oberfläche besitzen.
Der Maskenwulst und/oder das Stirnpolster und/oder nasal pillows sind bevorzugt keimabweisend und/oder selbstreinigend und/oder hautfreundlich und/oder hydrophob und/oder oleophob.

Der Maskenkörper ist auf seiner dem Patienten zugewandten Seite bevorzugt keimabweisend und/oder selbstreinigend und/oder hydrophob und/oder oleophob und/oder fotokatalytisch und/oder kratzfest und/oder beschlagfrei und/oder hautfreundlich. Der Maskenkörper ist auf seiner dem Patienten abgewandten Seite bevorzugt keimabweisend und/oder selbstreinigend und/oder hydrophob und/oder oleophob und/oder fotokatalytisch und/oder kratzfest. Insbesondere ist daran gedacht Einschnitte, Stege, Verdickungen oder Aussparungen im Bereich von Patienten-Interfaces keimabweisend und/oder selbstreinigend und/oder hydrophob und/oder oleophob und/oder fotokatalytisch und/oder kratzfest auszurüsten.
Auch ist daran gedacht Kontaktstellen der einzelnen Bauteile zueinander und/oder Kontaktstellen zum Patienten keimabweisend und/oder selbstreinigend und/oder hydrophob und/oder oleophob auszurüsten.
Besonders bevorzugt ist daran gedacht Patienten Interfaces im Bereich Ihrer der Luftströmung zugewandten Seite durch geeignete glatte Kunststoffe und/oder lackierte Oberflächen und/oder beschichtete Kunststoffe und/oder Oberflächen die eine Strukturierung im Nanometer- bis Mikrometer-Maßstab aufweisen derart auszurüsten, daß hierdurch eine reduzierte Reibung erzielt werden kann.

Die funktionale Eigenschaft der Oberfläche kann auch in einem Filter angewendet werden.
Die Filter sind auf der dem Patienten zugewandten Seite bevorzugt keimabweisend und/oder selbstreinigend und/oder hydrophob und/oder oleophob und/oder fotokatalytisch. Insbesondere ist auch daran gedacht Einschnitte, Stege, Verdickungen oder Aussparungen im Bereich der Fixierung eines Filters keimabweisend und/oder selbstreinigend und/oder hydrophob und/oder oleophob und/oder fotokatalytisch auszurüsten.
Auch ist daran gedacht Kontaktstellen zwischen Filter und Gerät und/oder Kontaktstellen zwischen Filter und Anwender keimabweisend und/oder selbstreinigend und/oder hydrophob und/oder oleophob und/oder fotokatalytisch auszurüsten. Durch die fotokatalytischen Eigenschaften werden anhaftende Teilchen und Mikroorganismen kalt verbrannt.
Bevorzugt ist daran gedacht Filter im Bereich Ihrer der Luftströmung zugewandten Seite durch geeignete glatte Kunststoffe und/oder lackierte Oberflächen und/oder beschichtete Kunststoffe und/oder Oberflächen die eine Strukturierung im Nanometerbis Mikrometermaßstab aufweisen derart auszurüsten, daß hierdurch eine reduzierte Reibung erzielt werden kann.

Bevorzugt ist ebenfalls daran gedacht sogenannte HME-Filter (Heat moisture exchange) derart auszurüsten, daß sie einen reduzierten Reibungswiderstand aufweisen und/oder keimabweisend und/oder selbstreinigend und/oder oleophob und/oder fotokatalytisch sind.

Funktionelle Oberflächen erweisen sich auch bei Schläuchen als vorteilhaft. Insbesondere bei Beatmungsgeräten, aber auch bei anderen medizintechnischen Geräten wie beispielsweise Absauggeräten werden vor allem im Bereich einer Verbindung zwischen Anwender/Patient mit dem Gerät Schläuche eingesetzt, um ein Medium zu leiten. Üblicherweise werden die Schläuche als Steckschläuche auswechselbar eingesetzt. Wird ein Schlauch nicht regelmäßig gereinigt und/oder ausgewechselt, so können Zurückgehaltene Schwebstoffe, Staubteile, Verunreinigungen Mikroorganismen den Durchströmungswiderstand heraufsetzen was dazu führt, daß die Leistung des Gerätes abnimmt beziehungsweise Verunreinigungen zum Patienten geleitet werden. Schläuche nach dem Stand der Technik müssen häufig gereinigt und/oder ausgetauscht werden, was zeit- und kostenintensiv ist. Die Reinigung muß häufig und gründlich erfolgen, um Kontaminationen effektiv zu entfernen.

Es ist daran gedacht, die Schläuche mit funktionalen Oberflächen auszurüsten. Dadurch wird eine längere Betriebsdauer der Schläuche bei einem gleichzeitig geringeren zeitlichen Aufwand für die Reinigung ermöglicht, was Kosten einspart.

Die Schläuche sind auf der dem Patienten/Anwender zugewandten Seite bevorzugt keimabweisend und/oder selbstreinigend und/oder hydrophob und/oder oleophob und/oder fotokatalytisch. Insbesondere ist auch daran gedacht Einschnitte, Stege, Verdickungen oder Aussparungen im Bereich der Fixierung eines Schlauches am Gerät und/oder am Patienten keimabweisend und/oder selbstreinigend und/oder hydrophob und/oder oleophob und/oder fotokatalytisch'auszurüsten.

Auch ist daran gedacht Kontaktstellen zwischen Schlauch und Gerät und/oder Kontaktstellen zwischen Schlauch und Anwender keimabweisend und/oder selbstreinigend und/oder hydrophob und/oder oleophob fotokatalytisch auszurüsten. Durch die fotokatalytischen Eigenschaften werden anhaftende Teilchen und Mikroorganismen kalt verbrannt.

Bevorzugt ist daran gedacht Schläuche im Bereich Ihrer der Luftströmung/Medienströmung zugewandten Seite durch geeignete glatte Kunststoffe und/oder lackierte Oberflächen und/oder beschichtete Kunststoffe und/oder Oberflächen die eine Strukturierung im Nanometer- bis Mikrometermaßstab aufweisen derart auszurüsten, daß hierdurch eine reduzierte Reibung erzielt werden kann. Bevorzugt ist ebenfalls daran gedacht, daß Schläuche eine elektrisch leitfähige Oberfläche aufweisen.

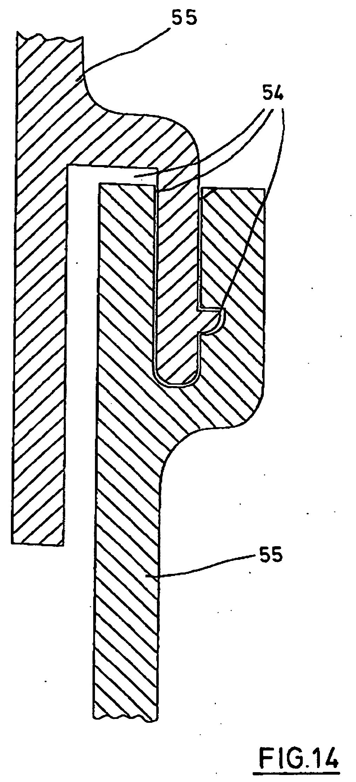

Fig. 14 zeigt eine beispielhafte Ausführungsform von Kontaktstellen (54) der erfindungsgemäße Formkörper (55) für medizinische Geräte. Bevorzugt weisen erfindungsgemäße Formkörper (55) für medizinische Geräte wenigstens abschnittsweise keimabweisend ausgebildete Kontaktstellen (54) auf. Die Kontaktstellen (54) sind beispielsweise wenigstens abschnittsweise durch Verringerung der Oberflächenadhäsion keimabweisend ausgebildet. Die Kontaktstellen sind beispielsweise wenigstens abschnittsweise durch Abtötung und/oder Wachstumshemmung von Mikroorganismen keimabweisend ausgebildet. Beispielsweise weisen die Kontaktstelle des Biozide auf. Die Kontaktstellen weisen wenigstens abschnittsweise bevorzugt als Wirkstoff Silber und/oder silberhaltige Verbindungen auf. In einem anderen Ausführungsbeispiel weist die Kontaktstelle als Wirkstoff ein antimikrobiell wirksames Polymer auf.

Die Kontaktstellen können alternativ und/oder zusätzlich wenigstens abschnittsweise hydrophil ausgebildet ist. Die Kontaktstellen können wenigstens abschnittsweise alternativ keimabweisend und/oder fotokatalytisch und/oder zusätzlich hydrophil ausgebildet sein. Ein Formkörper kann zumindest abschnittsweise eine kratzfeste Kontaktstelle aufweisen. Ein Formkörper kann bevorzugt eine keimabweisende, selbstreinigende und/oder hydrophile und/oder oleophobe und/oder reibungsarme und/oder leitende Kontaktstelle besitzen. Ebenfalls ist daran gedacht, daß die Kontaktstellen alle im Text erwähnten Eigenschaften entweder allein oder in Kombination zumindest abschnittsweise aufweisen können.

Diese modifizierten Formkörper für medizintechnische Geräte weisen adhäsionsverringernde Substanzen in einer solchen Menge auf, dass die Adhäsion von Mikroorganismen auf deren Oberfläche um mindestens 50% geringer ist als bei nicht-modifizierten Formkörpern, und/oder dass sie biozide Substanzen in einer solchen Menge aufweisen, dass mindestens 60% der verbleibenden Mikroorganismen innerhalb von 24 Stunden abgetötet werden.

Diese modifizierten Formkörper haben adhäsionsverringernde Substanzen inkorporiert. Ferner sind deren Oberflächen durch adhäsionsverringernde Substanzen modifiziert.

Außerdem sind biozide Substanzen inkorporiert, wobei als biozide Substanzen Silber, Silberionen freisetzende Substanzen, Kupfer, Kupferionen freisetzende Substanzen, Zink oder Zinkionen freisetzende Substanzen inkorporiert wurden.

Zur Herstellung modifizierter Formkörper werden die adhäsionsverringernden und bioziden Substanzen in die Kunststoffe eingebracht.

Ein Verfahren, z.B. biozide Materialien in die Kunststoffe einbringen, ist die Solvent Casting-Methode. Hierzu werden ausgehärteten Kunststoffe vermahlen, mit Bioziden vermischt und wieder in Form gepresst, ggf. unter Wärmeeinwirkung.
Das Biozid kann auch während des Kunststoff-Spritzvorgangs zugesetzt werden. Bei Abformmassen wird ein Biozid einer beliebigen Komponente der nicht-ausgehärteten Masse zuzugeben. Durch den Abbindevorgang wird dann ein Gummi erhalten, der biozide Eigenschaften aufweist.

Es kann aber auch während der Polymerisation zugeben werden oder während der Reaktion der Kreuzvernetzung. Schließlich kann Metall (z.B. Ag) mit Zeolith in Partikelform in der Polymerschmelze dispergieren und gemeinsam extrudiert werden. Alternativ können die adhäsionsverringernden Substanzen auf die Oberfläche der Kunststoffe aufgebracht und die bioziden Substanzen in die Kunststoffe inkorporiert werden.

Diese Kombinationen aus Antihaftfunktion und mikrobiozider Wirkung sind besonders vorteilhaft. So entstehen mikrobiozide Oberflächen von neuer Qualität, die folgende Eigenschaften aufweisen:
∘ Schichten mit niedriger Oberflächenenergie
∘ sehr glatte Oberflächen
∘ Bakterien haften nicht an und wachsen nicht
∘ mikrobiozide Oberflächen mit Schichten, die keimtötende Komponenten enthalten.

Beispielsweise weist zumindest ein Formkörper eines Sauerstoff-Zuschaltventils und/oder Anfeuchters und/oder Beatmungsgerätes und oder Patienteninterfaces und/oder einer Kopfhaube und/oder eines Schlauches und/oder eines Zubehörteiles wenigstens abschnittsweise eine erfindungsgemäße über die Oberfläche vermittelte Eigenschaft auf.

Formkörper, insbesondere als Geräteteil oder Bauteil oder Zubehörteil einer medizintechnischen Vorrichtung für Beatmung, Schlaftherapie, Herz und/oder Kreislauf-Therapie, Notfall oder Sauerstoff-Versorgung, einschliesslich der Diagnostik für alle vorgenannten Bereiche, dadurch gekennzeichnet, dass seine Oberfläche zumindest abschnittsweise eine funktionale Eigenschaft aufweist oder eine funktionale Eigenschaft vermittelt.

Formkörper mit zumindest den vorgenannten Merkmalen, wobei die funktionale Eigenschaft in keimabweisender, keimtötender oder keimvermindernder Wirkung besteht.

Formkörper mit zumindest den vorgenannten Merkmalen, wobei die funktionale Eigenschaft in antimikrobieller Wirkung besteht.

Formkörper mit zumindest den vorgenannten Merkmalen wobei die funktionale Eigenschaft in biozider Wirkung besteht.

Formkörper mit zumindest den vorgenannten Merkmalen, wobei die funktionale Eigenschaft in einer die Oberflächenadhäsion vermindernden Wirkung besteht.

Formkörper mit zumindest den vorgenannten Merkmalen, wobei die funktionale Eigenschaft in einer die Oberflächenenergie oder den Strömungswiderstand der Oberfläche oder in einer die Geräuschentwicklung an der Oberfläche strömender Flüssigkeiten oder Gasen senkenden Wirkung besteht.

Formkörper mit zumindest den vorgenannten Merkmalen, wobei die funktionale Eigenschaft in einer die Gleitreibung auf oder an der Oberfläche senkenden Wirkung besteht.

Formkörper mit zumindest den vorgenannten Merkmalen, wobei die funktionale Eigenschaft in einer die Benetzbarkeit oder die Beschlagsneigung verändernden Wirkung besteht.

Formkörper mit zumindest den vorgenannten Merkmalen, wobei die funktionale Eigenschaft in hydrophiler Wirkung besteht.

Formkörper mit zumindest den vorgenannten Merkmalen, wobei die funktionale Eigenschaft in oleophober Wirkung besteht.

Formkörper mit zumindest den vorgenannten Merkmalen, wobei die funktionale Eigenschaft in fotokatalytischer Wirkung besteht.

Formkörper mit zumindest den vorgenannten Merkmalen, wobei die funktionale Eigenschaft in elektrochemisch aktiver Wirkung besteht.

Formkörper mit zumindest den vorgenannten Merkmalen, wobei die funktionale Eigenschaft in einer die Kratzfestigkeit steigernden Wirkung besteht.

Formkörper mit zumindest den vorgenannten Merkmalen, wobei die funktionale Eigenschaft in einer die Selbstreinigungsfähigkeit steigernden Wirkung besteht.

Formkörper mit zumindest den vorgenannten Merkmalen, wobei an diesem wenigstens zwei der in den vorgenannten Merkmalen bezeichneten funktionalen Eigenschaften wenigstens bezüglich eines gleichen Teils oder Abschnitts der Oberfläche gleichzeitig realisiert werden.

Formkörper mit zumindest den vorgenannten Merkmalen, wobei die mehrfachen funktionalen Eigenschaften mit dem gleichen Material oder mit der gleichen Methode zur Oberflächengestaltung oder - behandlung oder einem analogen (physikalischen, chemischen oder biologischen) Wirkmechanismus erzielt werden.

Formkörper mit zumindest den vorgenannten Merkmalen, wobei die funktionale Eigenschaft oder mindestens eine der bezeichneten funktionalen Eigenschaften mit wenigstens zwei unterschiedlichen Materialien oder im Zusammenwirken von mindestens zwei unterschiedlichen Methoden zur Oberflächengestaltung oder -behandlung oder im Zusammenwirken von mindestens zwei verschiedenen (physikalischen, chemischen oder biologischen)
Wirkmechanismen erzielt oder verstärkt wird.

Formkörper mit zumindest den vorgenannten Merkmalen, wobei der Formkörper auch im Bereich der funktionalen Oberfläche zumindest abschnittsweise oder teilweise transparent ist.

Formkörper mit zumindest den vorgenannten Merkmalen, wobei gekennzeichnet, dass der Formkörper wenigstens abschnittsweise oder teilweise aus Kunststoff gefertigt, ist.

Formkörper mit zumindest den vorgenannten Merkmalen, wobei die Oberfläche des Formkörpers wenigstens abschnittsweise oder teilweise als Wirkstoff Silber oder silberhaltige Verbindungen enthält.

Formkörper mit zumindest den vorgenannten Merkmalen, wobei die Oberfläche des Formkörpers wenigstens abschnittsweise oder teilweise als Wirkstoff ein Polymer enthält.

Formkörper mit zumindest den vorgenannten Merkmalen, wobei die Oberfläche des Formkörpers mit einer Beschichtung versehen ist.

Formkörper mit zumindest den vorgenannten Merkmalen, wobei er wenigstens teilweise oder abschnittsweise eine Oberfläche mit einer Oberflächenenergie von weniger als 35 mN/m besitzt.

Formkörper mit zumindest den vorgenannten Merkmalen, wobei der Formkörper mindestens teilweise oder abschnittsweise eine strukturierte Oberfläche mit regelmässigen Erhebungen oder Vertiefungen aufweist.

Formkörper mit zumindest den vorgenannten Merkmalen, wobei der Formkörper mindestens teilweise oder abschnittsweise eine strukturierte Oberfläche mit unregelmässigen Erhebungen oder Vertiefungen aufweist.

Formkörper mit zumindest den vorgenannten Merkmalen, wobei der Formkörper mindestens teilweise oder abschnittsweise eine strukturierte Oberfläche sowohl mit regelmässigen wie mit unregelmässigen Erhebungen oder Vertiefungen aufweist.

Formkörper mit zumindest den vorgenannten Merkmalen, wobei die Erhebungen und/oder Vertiefungen eine Höhe bzw. Tiefe von 5 nm bis 200 [mu]m und/oder einen Abstand von 5 nm bis 200 [mu]m aufweisen.

Formkörper mit zumindest den vorgenannten Merkmalen, wobei die Erhebungen und/oder Vertiefungen eine Höhe bzw. Tiefe von 20 nm bis 25 [mu]m und/oder einen Abstand von 20 nm bis 25 [mu]m aufweisen.

Formkörper mit zumindest den vorgenannten Merkmalen, wobei die Erhebungen und/oder Vertiefungen eine Höhe bzw. Tiefe von 50 nm bis 4 [mu]m und/oder einen Abstand von 50 nm bis 4 [mu]m aufweisen.

Formkörper mit zumindest den vorgenannten Merkmalen, wobei der Formkörper eine Geräteschale, ein Schlauch, eine Gas oder Flüssigkeitsleitung oder -führung, ein Gas oder Flüssigkeitsbehälter, ein Patienteninterface, ein Zerstäuber, ein vernebler, ein Anfeuchter, ein Sauerstoffventil, ein Filter, ein Schalldämpfer, eine Absaugeinrichtung, ein Auffangbehälter oder ein Defibrillatorgehäuse, oder ein Bauteil, eine Komponente oder ein Zubehörteil (einschliesslich von Befestigungs, Transport und Aufbewahrungsvorrichtungen) zu einer der vorgenannten Vorrichtungen ist.
Formkörper mit zumindest den vorgenannten Merkmalen, wobei er zur Abformung oder Füllung einer medizintechnischen Vorrichtung nach Anspruch 1 dient.

Formkörper mit zumindest den vorgenannten Merkmalen, wobei durch die funktionale Eigenschaft die Keimbelastung bzw. die mikrobielle bzw. die biologische Belastung wenigstens hinsichtlich einer Belastungsart auf weniger als die Hälfte vermindert wird.
Formkörper mit zumindest den vorgenannten Merkmalen, wobei die funktionale Eigenschaft den zeitlichen Aufwand zur Reinigung hinsichtlich wenigstens einer Form von physikalischer oder chemischer Verschmutzung, Keimbelastung, mikrobieller Belastung oder biologischer Belastung vermindert wird, gemessen bei gleicher Reinigungsart an der Erreichung eines gleichen oder geringeren Vorgabewertes für eine der bezeichneten Verschmutzungen oder Belastungen.

Formkörper mit zumindest den vorgenannten Merkmalen, wobei die Verminderung des Zeitaufwandes zur Reinigung wenigstens 10% beträgt.

Verfahren zur Herstellung eines Formkörpers oder einer medizintechnischen Vorrichtung nach zumindest einem der vorgenannten Merkmale, wobei der Formkörper mittels KunststoffSpritzguss hergestellt und anschließend mindestens bereichsweise beschichtet wird.

Verfahren mit zumindest den vorgenannten Merkmalen, wobei Kunststoff vor oder während des Spritzgusses mit keimabweisenden Partikeln versehen wird.

Verfahren mit zumindest den vorgenannten Merkmalen, wobei wenigstens abschnittsweise die Oberfläche des Formkörpers nach dem Spritzguss mit keimabweisenden Partikeln versehen wird.

Verfahren mit zumindest den vorgenannten Merkmalen, wobei hydrophobe Partikel mit einem Durchmesser von 0,02 bis 100 [mu]m, vorzugsweise mit einem Durchmesser von 0,05 bis 30 [mu]m und/oder Partikel mit antimikrobiellen Eigenschaften mit einem Durchmesser von 20 bis 2000 [mu]m verwendet werden.

Verfahren mit zumindest den vorgenannten Merkmalen, wobei Mikropartikel mit einem Partikeldurchmesser von 0,02 bis 100 [mu]m, vorzugsweise von 0,1 bis 50 [mu]m, besonders bevorzugt von 0,1 bis 10 [mu]m verwendet werden.

Verfahren mit zumindest den vorgenannten Merkmalen, wobei als keimabweisende Partikel adhäsionsvermindernde Substanzen, Biozide, silberhaltige oder silberionenhaltige Substanzen und/oder antimikrobiell wirksame Polymere verwendet werden.

Verfahren mit zumindest den vorgenannten Merkmalen, wobei ein Partikel ausweisendes Lösungsmittel durch Aufsprühen, Aufrakeln, Auftropfen oder durch Eintauchen aufgebracht wird.

Verfahren mit zumindest den vorgenannten Merkmalen, wobei eine Hydrophilisierung der Oberfläche erfolgt.

Verfahren mit zumindest den vorgenannten Merkmalen, wobei die Partikel in die Oberfläche eingedrückt werden.

Verfahren mit zumindest den vorgenannten Merkmalen, wobei vor dem Spritzguss eine Form mit Partikeln versehen wird und die Partikel während des Spritzgusses eingedrückt werden.

Verfahren mit zumindest den vorgenannten Merkmalen, wobei die Oberfläche des Formkörpers wenigstens abschnittsweise mittels einer Abformung modifiziert wird.

Verfahren mit zumindest den vorgenannten Merkmalen, wobei die Abformung mittels des LIGA-Verfahrens erfolgt.

Beatmungsgerät mit einer Luftfördereinrichtung, an das über einen Atemgasschlauch eine Beatmungsmaske angeschlossen ist, wobei mindestens ein Bereich mindestens eines Bauteiles der Gesamteinrichtung, die aus dem Beatmungsgerät, dem Atemgasschlauch und der Beatmungsmaske besteht, eine Oberfläche aufweist, die keimabweisende und/oder biozide und/oder antimikrobielle Eigenschaften aufweist wobei ein Bauteil zumindest eines der vorgenannten Merkmale aufweisen kann.

## Patentansprüche

1. Formkörper, als Bauteil oder Zubehörteil eines Patienteninterface wobei der Formkörper wenigstens abschnittsweise aus Kunststoff gefertigt ist und wenigstens abschnittsweise eine Oberflächenbeschichtung mit zumindest einer fest verankerten Lage von Mikropartikeln aufweist, welche Erhebungen bilden, wodurch seine Oberfläche zumindest abschnittsweise eine funktionale Eigenschaft aufweist **dadurch gekennzeichnet, daß** der Formkörper der Maskenwulst (43) einer Beatmungsmaske (10) ist wobei der Maskenwulst (43) aus Silikon hergestellt ist und die Mikropartikel, die die Erhebungen auf der Oberfläche bilden, ausgewählt sind aus Silikaten, Mineralien, Metalloxiden, Metallpulvern, Kieselsäuren, Pigmenten oder Polymeren und die Oberflächenbeschichtung aus Silikon hergestellt ist.

2. Formkörper nach Anspruch 1 **dadurch gekennzeichnet, daß** der Formkörper mindestens teilweise oder abschnittsweise eine strukturierte Oberfläche mit regelmäßigen Erhebungen oder Vertiefungen aufweist.

3. Formkörper nach zumindest einem der Ansprüche 1 oder 2 **dadurch gekennzeichnet, daß** der Formkörper mindestens abschnittsweise eine strukturierte Oberfläche mit unregelmäßigen Erhebungen oder Vertiefungen aufweist.

4. Formkörper nach zumindest einem der Ansprüche 1-3 **dadurch gekennzeichnet, daß** die Erhebungen und/oder Vertiefungen eine Höhe bzw. Tiefe von 5 nm bis 200 µm und/oder einen Abstand von 5 nm bis 200 µm aufweisen.

5. Formkörper nach zumindest einem der vorhergehenden Ansprüche **dadurch gekennzeichnet, daß** er wenigstens teilweise oder abschnittsweise eine Oberfläche mit einer Oberflächenenergie von weniger als 35 mN/m besitzt.

6. Formkörper nach zumindest einem der vorhergehenden Ansprüche **dadurch gekennzeichnet, daß** die Mikropartikel eine unregelmässige Feinstruktur im Nanometerbereich an der Oberfläche aufweisen und einen Partikeldurchmesser von 0,02 bis 100 µm.

7. Formkörper nach zumindest einem der vorhergehenden Ansprüche **dadurch gekennzeichnet, daß** die strukturierte Oberfläche Erhebungen mit einer mittleren Höhe von 10 nm bis 200 µm und einem mittleren Abstand von 10 nm bis 200 µm aufweist und die äußere Form der Erhebungen eine mathematische Kurve und/oder Funktion mit einer Symmetrie bezüglich einer Ebene aufweisen.

8. Verfahren zur Herstellung eines Formkörpers oder einer medizintechnischen Vorrichtung nach zumindest einem der vorhergehendenden Ansprüche **dadurch gekennzeichnet, dass** der Formkörper mittels Kunststoffspritzguss hergestellt und anschliessend mindestens bereichsweise beschichtet wird.

9. Verfahren nach Anspruch 8 **dadurch gekennzeichnet, daß** zur Herstellung einer strukturierten, hydrophoben Oberfläche mit Erhebungen und Vertiefungen, eine organische Matrix, welche zumindest einen thermoplastischen, elastomeren oder duroplastischen Kunststoff aufweist mit einem Additiv versetzt wird, dass eine Partikelgrösse von 0,0001 bis 20 µm aufweist.

## Claims

1. A mold, as a component of or accessory for a patient interface, wherein the mold, at least in sections, is made of plastic and, at least in sections, has a surface coating with at least one firmly embedded layer of microparticles that form elevations, by means of which its surface, at least in sections, has a functional property **characterized in that** the mold of the mask cushion (43) is a ventilation mask (10) in which case the mask cushion (43) is made of silicone, and the microparticles that form the elevations on the surface are selected from silicates, minerals, metal oxides, metal powders, silicic acids, pigments or polymers, and the surface coating is made of silicone.

2. Mold according to claim 1 **characterized in that** the mold, at least partially or in sections, has a textured surface with regular elevations or depressions.

3. Mold according to at least one of the claims 1 or 2 **characterized in that** the mold, at least in sections, has a textured surface with irregular elevations or depressions.

4. Mold according to at least one of the claims 1-3 **characterized in that** the elevations and/or depressions have a height or depth of 5 nm to 200 µm and/or a distance of 5 nm to 200 µm.

5. Mold according to at least one of the preceding claims **characterized in that** it, at least partially or in sections, has a surface with a surface energy of less than 35 mN/m.

6. Mold according to at least one of the preceding claims **characterized in that** the microparticles have an irregular fine structure in the nanometer range on the surface and a particle diameter of 0,02 to 100 µm.

7. Mold according to at least one of the preceding claims **characterized in that** the textured surface has elevations with a mean height of 10 nm to 200 µm and a mean distance of 10 nm to 200 µm, and the exterior shape of the elevations has a mathematical curve and/or function with a symmetry with respect to a plane.

8. Process for the manufacturing of a mold or a medical technology device according to at least one of the preceding claims **characterized in that** the mold is manufactured by means of plastic injection molding and then coated, at least in some areas.

9. Process according to claim 8 **characterized in that**, in order to produce a textured, hydrophobic surface with elevations and depressions, an organic matrix, which has at least one thermoplastic, elastomeric, or duroplastic plastic, is mixed with an additive that has a particle size of 0,0001 to 20 µm.

## Revendications

1. Corps moulé en guise d'accessoire d'une interface patient, au moins partiellement fabriqué en plastique et présentant au moins partiellement un revêtement de surface doté au minimum d'une couche fermement ancrée de microparticules formant des surélévations, de sorte que sa surface présente au moins partiellement une propriété fonctionnelle **caractérisée par le fait que** le corps moulé est la jupe (43) d'un masque respiratoire (10), la jupe du masque (43) étant fabriquée en silicone et les microparticules qui forment les surélévations sur la surface étant sélectionnées parmi les silicates, minéraux, oxydes métalliques, poudres métalliques, silices, pigments ou polymères et le revêtement de surface étant fabriqué en silicone.

2. Corps moulé selon la revendication 1 **caractérisé en ce que** le corps moulé présente au moins partiellement une surface structurée avec surélévations ou renfoncements réguliers.

3. Corps moulé selon au moins l'une des revendications 1 ou 2 **caractérisé en ce que** le corps moulé présente au moins partiellement une surface structurée avec surélévations ou renfoncements irréguliers.

4. Corps moulé selon au moins l'une des revendications 1-3 **caractérisé en ce que** les surélévations et/ou renfoncements présentent une hauteur ou une profondeur de 5 nm à 200 µm et un écartement de 5 nm à 200 µm.

5. Corps moulé selon au moins l'une des revendications précédentes **caractérisé en ce qu'**il possède au moins partiellement une surface présentant une énergie de surface inférieure à 35 mN/m.

6. Corps moulé selon au moins l'une des revendications précédentes **caractérisé en ce que** les microparticules présentent une structure fine irrégulière de l'ordre du nanomètre sur la surface et une taille des particules de 0,02 à 100 µm.

7. Corps moulé selon au moins l'une des revendications précédentes **caractérisé en ce que** la surface structurée présente des surélévations d'une hauteur moyenne de 10 nm à 200 µm et écartées en moyenne de 10 nm à 200 µm et **en ce que** la forme extérieure des surélévations présentent une courbe mathématique et/ou une fonction mathématique avec une symétrie par rapport à un plan.

8. Procédé de fabrication d'un corps moulé ou d'un dispositif médical selon au moins l'une des revendications précédentes **caractérisé en ce que** le corps moulé est fabriqué par moulage par injection et ensuite revêtu au moins par zones.

9. Procédé selon la revendication 8 **caractérisé en ce que** pour la fabrication d'une surface structurée hydrophobe avec surélévations et renfoncements on additionne à une matrice organique, laquelle présente au moins une matière synthétique thermoplastique, élastomère ou thermodurcissable, un additif dont la taille des particules est de 0,0001 à 20 µm.
